# EUROPEAN PATENT APPLICATION

(11) **EP 2 682 119 A1**
(43) Date of publication of application: **08.01.2014**
(21) Application number: 12174723.2
(22) Date of filing: 03.07.2012
(51) Int. Cl.: A61K 31/404, A61K 31/4045, A61K 31/405, A61K 31/416, A61K 31/4184, A61K 31/47, A61P 25/00, A61P 29/00

(54) **Aromatic N-heterocycle derivatives for use as medicine**

(71) Applicant: Université Libre de Bruxelles, 1050 Bruxelles (BE)
(72) Inventor: Soubhye, Jalal, 1050 Bruxelles (BE); Dufrasne, François, 1180 Bruxelles (BE); Zouaoui Boudjeltia, Karim, 6540 Lobbes (BE); van Antwerpen, Pierre, 1090 Bruxelles (BE)
(74) Representative: Pecher, Nicolas

(57) **Abstract**

The invention relates to compounds, or a pharmaceutically acceptable salt, solvate, hydrate, polymorph, co-crystal thereof, wherein said compound is of formula A-B (I) wherein A is substitued or not aromatic N-heterocycle; and B is -[C(R¹)(R²)]ₙ-R³ wherein n is an integer between 1 and 10, R¹ and R² represent independently in each of the n units a substituent selected from the group consisting of hydrogen, substituted or not C₁-C₁₀ alkyl, halogen, oxy derivatives, thioderivatives, substituted or not C₃-C₁₀ cycloalkyl, substituted or not C₆-C₁₈ aryl, substituted or not C₂-C₁₀ alkenyl, substituted or not C₂-C₁₀ alkynyl, R³ represents a substituent selected from the group consisting of hydrogen, substituted or not C₁-C₁₀ alkyl, halogen, substituted or not C₃-C₁₀ cycloalkyl, substituted or not C₆-C₁₈ aryl, substituted or not C₂-C₁₀ alkenyl, substituted or not C₂-C₁₀ alkynyl, amino, heterocycle, thiocyanate, thio derivatives, hydroxylamine, amido, sulfonamide, amino sulfone derivatives, ether, thioether, ester, thioester, azido, azo, cyano, nitro, nitrooxy, carbamate, thiocarbamate, sulfinyl derivative, sulfonyl derivatives, acyl derivatives, oxy derivatives, ureido, thioureido, imino, oximino, hydrazino, thioamido, carboxylic, phosphate, phosphonate, carbamoyl phosphate; with the proviso that when A is 5-fluoroindole, B is bonded at position 3 of the 5-fluoroindole moiety, R¹ is hydrogen and n is between 2 and 10, R³ is not -NR⁴R⁵ wherein R⁴ and R⁵ independently represent a substituent selected from the group consisting of hydrogen, C₁-C₁₀ alkyl, C₃-C₁₀ cycloalkyl, aminoalkyl, or R⁴ and R⁵ taken together with the nitrogen atom to which they are attached to form a four to ten-membered heterocycle, and R² is not independently in each of the n units a substituent selected from the group consisting of hydrogen, C₁-C₁₀ alkyl, halogen, alkoxy, aminoalkyl, and alkylamino; for the treatment or the prophylaxis of neurologic and/or inflammatory diseases or disorders. The present invention also provides pharmaceutical composition and in vitro method for inhibiting myeloperoxidase enzyme activity or low density lipoproteins oxidation.

## Description

### Field of the invention

The invention relates to aromatic N-heterocycle derivatives for use as medicine. In particular, the invention refers to aromatic N-heterocycle derivatives for use in the treatment or the prophylaxis of neurologic and/or inflammatory diseases or disorders.

### Background of the invention

Myeloperoxidase (EC 1.11.2.2), MPO, is a heme-containing enzyme found in mammalian neutrophils and stored in a large amount in azurophilic granules of these cells. It plays a fundamental role in antimicrobial and antiviral system. Upon phagocytosis of pathogen, azurophilic granules discharge their content into the forming phagosomes, where MPO is activated. The microbicidal activity of MPO is due to its ability to oxidation of halide ions and pseudohalide by H₂O₂ to produce their respective hypohalous acids. However, MPO can be released in the outside of phagocytes, and the reactive species produced by MPO also contribute to tissue damage by oxidizing many types of biomolecules (lipids, proteins, DNA, etc.). Active MPO was detected in arteriosclerotic plaques, in brain tissue showing Alzheimer-type pathology or Parkinson's disease, in multiple sclerosis lesions, in synovial fluids of patients with rheumatoid arthritis, in glomerular basement membrane in membranous glomerulonephritis, and in PMN-mediated liver diseases. Enhanced plasma levels of MPO are regarded as a risk factor of coronary artery disease. These finding make MPO implicated in many chronic inflammatory diseases.

Lipids have a central role in the pathogenesis of plaques. Observational data support a strong association between plasma lipid levels and the risk of cardiovascular diseases. The role of lipids, especially low-density lipoproteins LDL, in formation and evolution of atheromatous plaque has been largely documented showing that the oxidative modification of LDL is an important step in the process. It has been demonstrated that the oxidized LDL can induce the formation of foam cells and of a number of potentially pro-atherogenic metabolites such as pro-inflammatory cytokines and chemokines in monocytes, endothelial cells, and smooth muscle cells. As a consequence, oxidized LDL accumulates in the vascular cell wall and promotes a local inflammatory process. The key role of MPO in this process has been documented and the enzyme has been involved in the oxidative modification of apolipoprotein B-100 in the intima and at the surface of endothelial cells. Furthermore, high-density lipoprotein HDL, which is the major carrier of lipid hydroperoxides in circulating plasma, can be a target of oxidative species produced by MPO. It has been also reported that MPO selectively binds to apolipoprotein B-100 which is the major apolipoprotein in LDL. MPO therefore appears as a crucial pharmacological target. Its inhibition is mainly focused on either the scavenging of the oxygen species produced by MPO or on a direct inhibition of the synthesis of HOCI.

WO 2010/136546 discloses 3-alkyl-5-fluoroindole derivatives as myeloperoxidase inhibitors, having middle inhibiting character varying in function of the carbon chain length. These compounds, however, showed strong affinity with serotonine receptors. Such strong affinity between 3-alkyl-5-fluoroindole derivatives and serotonine receptors 5-HT receptors may cause adverse effects at the level of the central nervous system or the cardiovascular system. For example, disrupting serotonin receptor activity can lead to major depressive disorder, drownsiness, somnipathy, sleep disorder, vaso-dilatation or vaso-constriction leading to hypotension or hypertension.

There is a need for new compounds having enhanced inhibiting character. In particular, there is a need for new compounds showing lower affinity to serotonin receptors.

### Summary of the invention

The present invention addresses at least partly drawbacks of the prior art. The applicant surprisingly found new aromatic N-heterocycle derivatives showing excellent inhibition properties towards myeloperoxidase enzyme. Such derivatives may also exhibit excellent inhition properties towards LDL oxidation carried out by MPO. The compounds of the present invention may show lower interaction or affinity with serotonin receptors.

In a first aspect, the present invention provides compounds, or a pharmaceutically acceptable salt, solvate, hydrate, polymorph, co-crystal thereof, wherein said compound is of formula A-B (I) wherein
A is substitued or not aromatic N-heterocycle; and
B is -[C(R¹)(R²)ₙ-R³ wherein
n is an integer between 1 and 10,
R¹ and R² represent independently in each of the n units a substituent selected from the group consisting of hydrogen, substituted or not C₁-C₁₀ alkyl, halogen, oxy derivatives, thio derivatives, substituted or not C₃-C₁₀ cycloalkyl, substituted or not C₆-C₁₈ aryl, subtituted or not C₂-C₁₀ alkenyl, and subtituted or not C₂-C₁₀ alkynyl;
R³ represents a substituent selected from the group consisting of hydrogen, substituted or not C₁-C₁₀ alkyl, halogen, substituted or not C₃-C₁₀ cycloalkyl, substituted or not C₆-C₁₈ aryl, subtituted or not C₂-C₁₀ alkenyl, and subtituted or not C₂-C₁₀ alkynyl, amino, heterocycle, thiocyanate, thio derivatives, hydroxylamine, amido, sulfonamide, amino sulfone derivatives, ether, thioether, ester, thioester, azido, azo, cyano, nitro, nitrooxy, carbamate, thiocarbamate, sulfinyl derivative, sulfonyl derivatives, acyl derivatives, oxy derivatives, ureido, thioureido, imino, oximino, hydrazino, thioamido, carboxylic, phosphate, phosphonate, carbamoyl phosphate;
with the proviso that when A is 5-fluoroindole, B is bonded at position 3 of the 5-fluoroindole moiety, R¹ is hydrogen and n is between 2 and 10, R³ is not -NR⁴R⁵ wherein R⁴ and R⁵ independently represent a substituent selected from the group consisting of hydrogen, C₁-C₁₀ alkyl, C₃-C₁₀ cycloalkyl, aminoalkyl, or R⁴ and R⁵ taken together with the nitrogen atom to which they are attached to form a four to ten-membered heterocycle, and R² is not independently in each of the n units a substituent selected from the group consisting of hydrogen, C₁-C₁₀ alkyl, halogen, alkoxy, aminoalkyl and alkylamino;
for the treatment or the prophylaxis of neurologic and/or inflammatory diseases or disorders.

The applicant showed that compounds, such as in particular 3-substituted-5-fluoroindole or 7-fluoroindole derivatives according to the present invention, strongly inhibit myeloperoxidase enzyme. Similar effect may also be obtained with other compounds wherein the aromatic N-heterocyle is different from 5-fluoroindole or 7-fluoroindole derivatives. The low IC₅₀ values observed against myeloperoxidase enzyme (MPO) for said compounds of the present invention allow their therapeutic use in diseases in which a decrease of MPO activity is beneficial. Compounds of the present invention may also inhibit LDL oxidation. Furthermore, compounds of the present invention may have limited interaction with serotonin receptor which may be beneficial for therapeutic treatment.

In a further aspect of the present invention, a pharmaceutical composition is provided. In particular, said pharmaceutical composition comprises a therapeutically effective amount of a compound according to the present invention, or a pharmaceutically acceptable salt thereof, in admixture with a pharmaceutically acceptable adjuvant, diluent or carrier. Said pharmaceutical composition may be used for the treatment or prophylaxis of neurologic and/or inflammatory diseases or disorders.

In another aspect, the present invention provides a method for the treatment of atherosclerosis or rheumatic diseases. In yet another aspect, the present invention provides an in vitro method for inhibiting oxidation of low density lipoproteins carried out by myeloperoxidase enzyme.

In another aspect, compounds of the present invention may be used as medicine.

### Detailed description of the invention

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

As used herein, the singular forms "a", "an", and "the" include both singular and plural referents unless the context clearly dictates otherwise. The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of". The recitation of numerical ranges by endpoints includes all numbers and fractions subsumed within the respective ranges, as well as the recited endpoints. The term "about" as used herein when referring to a measurable value such as a parameter, an amount, a temporal duration, and the like, is meant to encompass variations of +/-10% or less, preferably +/-5% or less, more preferably or less, and still more preferably +/-0.1 % or less of and from the specified value, insofar such variations are appropriate to perform in the disclosed invention. It is to be understood that the value to which the modifier "about" refers is itself also specifically, and preferably, disclosed. All documents cited in the present specification are hereby incorporated by reference in their entirety.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Whenever the term "substituted" is used in the present invention, it is meant to indicate that one or more hydrogen on the atom indicated in the expression using "substituted" is replaced with a selection from the indicated group, provided that the indicated atom's normal valence is not exceeded, and that the substitution results in a chemically stable compound, i.e. a compound that is sufficiently robust to survive isolation to a useful degree of purity from a reaction mixture, and formulation into a therapeutic agent. Substituents may be selected from, but not limited to, the group comprising alkyl, cycloalkyl, aryl, halogen, hydroxyl, amino, heterocycle, thiocyanate, thio derivative, hydroxylamine, amido, sulfonamide, amino sulfone derivatives, ether, thioether, ester, thioester, thiol, azido, cyano, nitro, nitrooxy, carbamate, thiocarbamate, sulfinyl derivative, sulfonyl derivative, alkenyl, alkynyl, acyl derivative, oxy derivative, ureido, thioureido, imino, oximino, hydrazino, thioamido, carboxylic, phosphate, phosphonate, carbamoyl phosphate.

The term "alkyl" by itself or as part of another substituent refers to a hydrocarbyl radical of formula CₙH₂ₙ₊₁ wherein n is a number greater than or equal to 1. Generally, alkyl groups of the present invention comprise from 1 to 10 carbon atoms, preferably from 1 to 6 carbon atoms, more preferably from 1 to 4 carbon atoms. The term "alkyl" encompasses linear or branched alkyl. The term "alkyl" may encompass alkyl groups substituted or not by one or more substituents as defined above. When a subscript is used herein following a carbon atom, the subscript refers to the number of carbon atoms that the named group may contain. Thus, for example, C₁₋₄alkyl means an alkyl of one to four carbon atoms. For example, the "C₁-C₁₀ alkyl" refers but is not limited to methyl, ethyl, n-propyl, i-propyl, n-butyl, i-butyl, s-butyl, t-butyl, 1-pentyl, 2-pentyl, 3-pentyl, i-pentyl, neo-pentyl, t-pentyl, 1-hexyl, 2-hexyl, 3-hexyl, 1-methyl-1-ethyl-n-pentyl, 1,1,2-tri-methyl-n-propyl, 1,2,2-trimethyl-n-propyl, 3,3-dimethyl-n-butyl, 1-heptyl, 2-heptyl, 1-ethyl-1,2-dimethyl-n-propyl, 1-ethyl-2,2-dimethyl-n-propyl, 1-octyl, 3-octyl, 4-methyl-3-n-heptyl, 6-methyl-2-n-heptyl, 2-propyl-1-n-heptyl, 2,4,4-trimethyl-1-n-pentyl, 1-nonyl, 2-nonyl, 2,6-dimethyl-4-n-heptyl, 3-ethyl-2,2-dimethyl-3-n-pentyl, 3,5,5-trimethyl-1-n-hexyl, 1-decyl, 2-decyl, 4-decyl, 3,7-dimethyl-1-n-octyl, 3,7-dimethyl-3-n-octyl. For example, the term "C₁-C₆ alkyl" refers to, but is not limited to, methyl, ethyl, n-propyl, i-propyl, n-butyl, i-butyl, s-butyl, t-butyl, 1-pentyl, 2-pentyl, 3-pentyl, i-pentyl, neo-pentyl, t-pentyl, 1-hexyl, 2-hexyl, 3-hexyl, 1-methyl-1-ethyl-n-pentyl, 1,1,2-tri-methyl-n-propyl, 1,2,2-trimethyl-n-propyl, 3,3-dimethyl-n-butyl.

The term "four to ten-membered heterocycle" as used herein refers to a four to ten-membered carbocyclic ring structure interrupted by at least one nitrogen atom. Preferably, said heterocycle is four to eight-membered heterocycle, more preferably five to six-membered heterocycle. The "four to ten-membered heterocycle" may optionally be substituted by one or more substituent(s) as defined above, may further comprise another heteroatom such as sulfur, oxygen, phosphorus or nitrogen, or may be aromatic. Non-limiting examples of "four to ten-membered heterocycle" may be azetidine, pyrrolidine, piperidine, piperazine, azepane, azocane, methylpiperidine, pyrrole, indole, isoindole, pyridine, triazinane, triazine, azocine, azaphosphinane, morpholine, thiomorpholine, oxazinane, thiazinane, azaphosphinine, thiazine, or oxazine.

The term "aryl" as used herein refers to a polyunsaturated, aromatic hydrocarbyl group having a single ring (i.e. phenyl) or multiple aromatic rings fused together (e.g. naphtyl) or linked covalently, typically containing 6 to 18 carbon atoms, wherein at least one ring is aromatic. Non-limiting examples of aryl comprise phenyl, biphenylyl, biphenylenyl, tetralinyl, azulenyl, naphthalenyl, indenyl, acenaphtenyl, phenanthryl, indanyl, pyrenyl. The term "aryl" encompasses aryl substituted by one or more substituent(s) as defined above.

The term "halogen" as used herein refers to F, Cl, Br, or I.

The term "cycloalkyl" as used herein is a cyclic alkyl group, that is to say, a monovalent, saturated, or unsaturated hydrocarbyl group having 1 or 2 cyclic structure. Cycloalkyl includes all saturated hydrocarbon groups containing 1 to 2 rings, including monocyclic or bicyclic groups. Cycloalkyl groups may comprise 3 or more carbon atoms in the ring and generally, according to this invention comprise from 3 to 10 carbon atoms. The term "C₃-C₁₀ cycloalkyl" as used herein refers to a cycloalkyl groups comprising from 3 to 10 carbon atoms and encompasses substitued cycloalkyl groups bearing a substituent as defined above. For example, the term may include cyclopropane, cyclobutane, cyclopentane, cyclohexane, cycloheptane, cyclooctane, cyclononane, or cyclodecane.

The term "heterocycle", as used herein as a substituent is defined as including an aromatic or non aromatic cyclic alkyl, alkenyl, aryl or alkynyl moiety as defined above, having at least one O, S, P and/or N atom interrupting the carbocyclic ring structure and optionally, one of the carbon of the carbocyclic ring structure may be replaced by a carbonyl. Non-limiting examples of aromatic heterocycles are pyridyl, furyl, pyrrolyl, thienyl, isothiazolyl, imidazolyl, benzimidazolyl, tetrazolyl, quinazolinyl, quinolizinyl, naphthyridinyl, pyridazinyl, pyrimidinyl, pyrazinyl, quinolyl, isoquinolyl, isobenzofuranyl, benzothienyl, pyrazolyl, indolyl, indolizinyl, purinyl, isoindolyl, carbazolyl, thiazolyl, 1, 2, 4-thiadiazolyl, thieno (2,3-b) furanyl, furopyranyl, benzofuranyl, benzoxepinyl, isooxazolyl, oxazolyl, thianthrenyl, benzothiazolyl, or benzoxazolyl, cinnolinyl, phthalazinyl, quinoxalinyl, phenanthridinyl, acridinyl, perimidinyl, phenanthrolinyl, phenothiazinyl, furazanyl, isochromanyl, indolinyl, xanthenyl, hypoxanthinyl, pteridinyl, 5-azacytidinyl, 5-azauracilyl, triazolopyridinyl, imidazolopyridinyl, pyrrolopyrimidinyl, and pyrazolopyrimidinyl optionally substituted by alkyl or as described above for the alkyl groups. Non-limiting examples of non aromatic heterocycles are tetrahydrofuranyl, tetrahydropyranyl, piperidinyl, piperidyl, piperazinyl, imidazolidinyl, morpholino, morpholinyl, 1-oxaspiro(4,5)dec-2-yl, pyrrolidinyl, 2-oxo-pyrrolidinyl, sugar moieties (i.e. glucose, pentose, hexose, ribose, fructose, which may also be substituted) or the same which can optionally be substituted with any suitable group. The term "heterocycle" also includes bicyclic, tricyclic and tetracyclic, spiro groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring or where a monocyclic heterocyclic group is bridged by an alkylene group, such as quinuclidinyl, 7-azabicyclo (2,2,1) heptanyl, 7-oxabicyclo (2,2,1) heptanyl, 8-azabicyclo (3,2,1) octanyl. The term "heterocycle" also encompasses substituted heterocycle as defined above, bearing one or more substituent as defined above.

The term "nitro" as used herein refers to the group -NO₂.

The term "cyano" as used herein refers to the group -CN.

The term "hydroxyl" as used herein refers to the group -OH.

The term "thiol" as used herein refers to the group -SH.

The term "carboxylic" as used herein refers to the group -CO₂H.

The term "amido" as used herein refers to the group -C(O)-NR^{a}R^{b} wherein R^{a} and R^{b} independently represents hydrogen, substituted or not C₃-C₁₀ cycloalkyl, substituted or not C₆-C₁₈ aryl, substituted or not C₁-C₁₅ alkyl, substituted or not C₃-C₁₀ heterocycle, substituted or not C₂-C₁₅ alkenyl, substituted or not C₂-C₁₅ alkynyl, or R^{a} and R^{b} are taken together with the nitrogen atom to which they are attached to form a four to ten membered heterocycle.

The term "ester" refers to the group -C(O)-O-R^{c} or -O-C(O)-R^{c} wherein R^{c} represents a moiety selected from the group consisting of hydrogen, substituted or not C₃-C₁₀ cycloalkyl, substituted or not C₆-C₁₈ aryl, substituted or not C₁-C₁₅ alkyl, substituted or not C₃-C₁₀ heterocycle, substituted or not C₂-C₁₅ alkenyl, substituted or not C₂-C₁₅ alkynyl.

The term "thioester" refers to the group -C(S)-O-R^{c}, -C(O)-S-R^{c}, -S-C(O)-R^{c} or -O-C(S)-R^{c} wherein R^{c} is as defined above.

The term "oxy derivative", as used herein is defined as including -O-R^{c} groups wherein R^{c} is as defined above. Non-limiting examples are alkoxy, alkenyloxy, alkynyloxy, acyloxy, aryloxy, aralkoxy or heterocyclooxy such as pentyloxy, allyloxy, methoxy, ethoxy,-phenoxy, benzyloxy, 2-naphthyloxy, 2-pyridyloxy, methylenedioxy. In particular, the term "alkoxy" is a oxy derivative wherein R^{c} is substituted or not C₁-C₁₀ alkyl.

The term "amino" by itself or as part of another substituent refers to a group of formula -N(R^{a})(R^{b}) wherein R^{a} and R^{b} are as defined above. Alkylamino include mono-lower alkyl amino group (e.g. mono C₁₋₆alkylamino group such as methylamino and ethylamino), di-lower alkylamino group (e.g. di-C₁₋₆alkylamino group such as dimethylamino and diethylamino). Non-limiting examples of suitable alkylamino groups also include n-propylamino, isopropylamino, n-butylamino, isobutylamino, sec-butylamino, tert-butylamino, pentylamino, n-hexylamino, di-n-propylamino, diisopropylamino, ethylmethylamino, methyl-n-propylamino, methyl-i-propylamino, n-butylmethylamino, i-butylmethylamino, t-butylmethylamino, ethyl-n-propylamino, ethyl-i-propylamino, n-butylethylamino, i-butylethylamino, t-butylethylamino, di-n-butylamino, di-i-butylamino, methylpentylamino, methylhexylamino, ethylpentylamino, ethylhexylamino, propylpentylamino, propylhexylamino, and the like.

The term "thio derivative", as used herein is defined as including -S-R^{c} groups wherein R^{c} is as defined above. In particular, the term "thioalkyl" refers to a thio derivative wherein R^{c} is substituted or not C₁-C₁₀ alkyl. For example, "thioalkyl" encompasses -S-(CH₂)₂-NH₂.

The term "nitrooxy", as used herein, represents a group of the formula-ONO₂.

The term "azido", as used herein, represents a group of the formula -N₃.

The term "sulfonamide", as used herein, represents a group of the formula -SO₂NR^{a}R^{b} wherein R^{a} and R^{b} are as defined above.

The term "ether" is defined as including a group selected from C₁-C₅₀ straight or branched alkyl, or C₂-C₅₀ straight or branched alkenyl or alkynyl groups or a combination of the same, interrupted by one or more oxygen atoms.

The term "thioether" is defined as including a group selected from C₁-C₅₀ straight or branched alkyl, or C₂-C₅₀ straight or branched alkenyl or alkynyl groups or a combination of the same, interrupted by one or more sulfur atoms.

The term "sulfonyl derivative" as used herein, is defined as including a group of the formula -SO₂-R^{c}, wherein R^{c} is as defined above. Non limiting examples are alkylsulfonyl, alkenylsulfonyl, alkynylsulfonyl and arylsulfonyl.

The term "sulfinyl derivative" as used herein, is defined as including a group of the formula -SO-R^{c}, wherein R^{c} is as defined above. Non limiting examples are alkylsulfinyl, alkenylsulfinyl, alkynylsulfinyl and arylsulfinyl.

The term "alkenyl" as used herein, is defined as including both branched and unbranched, unsaturated hydrocarbon radicals having at least one double bond such as ethenyl (= vinyl), 1methyl-1-ethenyl, 2, 2-dimethyl-1-ethenyl, 1-propenyl, 2-propenyl (₌ allyl), 1-butenyl, 2-butenyl, 3-butenyl, 4-pentenyl, 1-methyl-4-pentenyl, 3-methyl-l-pentenyl, 1-hexenyl, 2-hexenyl, and the like and being optionally substituted by at least one substituent as defined above.

The term "alkynyl" as used herein, is defined as including a monovalent branched or unbranched hydrocarbon radical containing at least one carbon-carbon triple bond, for example ethynyl, 2-propynyl (= propargyl), and the like and being optionally substituted by at least one substituent as defined above.

The term "acyl derivative" as used herein, represents a radical of the formula -CO-R^{c} wherein R^{c} is as defined above and may also be hydrogen. Non-limiting examples are formyl, acetyl, propionyl, isobutyryl, valeryl, lauroyl, heptanedioyl, cyclohexanecarbonyl, crotonoyl, fumaroyl, acryloyl, benzoyl, naphthoyl, furoyl, nicotinoyl, 4-carboxybutanoyl, oxalyl, ethoxalyl, cysteinyl, oxamoyl.

The term "ureido" as used herein refers to a group of formula -N(R^{c})-C(O)-NR^{a}R^{b} wherein R^{c}, R^{a}, R^{b} are as defined above.

The term "thioureido" as used herein refers to a group of formula-N(R^{c})-C(S)-NR^{a}R^{b} wherein R^{c}, R^{a}, R^{b} are as defined above.

The term "imino" as used herein refers to a group of formula -C(R^{c})=N-R^{d} or -N=C(R^{a})(R^{b}) wherein R^{a}, R^{b}, R^{c} are as defined above and R^{d} represents a moiety selected from the group consisting of hydrogen, C₃-C₁₀ cycloalkyl, C₆-C₁₈ aryl, C₁-C₁₅ alkyl, C₃-C₁₀ heterocycle, C₂-C₁₅ alkenyl, C₂-C₁₅ alkynyl.

The term "oximino" as used herein refers to a group of formula-C(R^{c})=N-OH wherein R^{c} is as defined above.

The term "hydrazino" as used herein refers to a group of formula-N(R^{c})-NR^{b}R^{a} wherein R^{a}, R^{b}, R^{c} are as defined above.

The term "thioamido" as used herein refers to a group of formula -C(S)-NR^{a}R^{b} wherein R^{a} and R^{b} are as defined above.

The term "phosphate" as used herein refers to a group of formula -OP(O)-OR^{c}OR^{d} wherein R^{c} and R^{d} are as defined above.

The term "phosphonate" as used herein refers to a group of formula-P(O)-OR^{d}OR^{c} wherein R^{c} and R^{d} are as defined above.

The term "carbamoyl phosphate" as used herein refers to a group of formula -N(R^{c})-C(O)-O-P(O)(OR^{d}) (OR^{e}) wherein R^{c}, R^{d} are as defined above and R^{e} represents a moiety selected from the group consisting of hydrogen, C₃-C₁₀ cycloalkyl, C₆-C₁₈ aryl, C₁-C₁₀ alkyl, C₃-C₁₀ heterocycle, C₂-C₁₀ alkenyl, C₂-C₁₀ alkynyl.

The term "thiocyanate" as used herein refers to a group of formula-SCN.

The term "hydroxylamine" as used herein refers to a group of formula-N(OH)R^{c} wherein R^{c} is as defined above.

The term "amino sulfone derivatives" as used herein refers to a group of formula -N(R^{c})SO₂-R^{d} wherein R^{d} and R^{c} are as defined above.

The term "carbamate" as used herein refers to a group of formula - N(R^{c})-C(O)-OR^{d} wherein R^{d} and R^{c} are as defined above.

The term "thiocarbamate" as used herein refers to a group of formula-N(R^{c})-C(S)-OR^{d} wherein R^{d} and R^{c} are as defined above.

The term "IC₅₀" as used herein refers to the half maximal inhibitory concentration. IC₅₀ represents the concentration of a compound that is required for 50% inhibition of an enzyme *in vitro.* In the context of the present invention, IC₅₀ values were determined against myeloperoxidase enzyme.

The term "n unit" as used herein refers to the group -(CR¹R²)-. Hence, the term "R¹ and R² represents independently in each of the n units a substituent" as used herein means that in each "n unit", i.e. in each -(C(R¹)(R²)) - group, R¹ and R² may be one of the cited substituents, independently of the other or adjacent "n unit" contained in said compound.

In a first aspect, the present invention relates to compounds, or a pharmaceutically acceptable salt, solvate, hydrate, polymorph, co-crystal thereof, wherein said compound is of formula A-B (I) wherein
A is substitued or not aromatic N-heterocycle; and
B is -[C(R¹)(R²)]ₙ-R³ wherein
n is an integer between 1 and 10,
R¹ and R² represent independently in each of the n units a substituent selected from the group consisting of hydrogen, substituted or not C₁-C₁₀ alkyl, halogen, oxy derivatives, thio derivatives, substituted or not C₃-C₁₀ cycloalkyl, substituted or not C₆-C₁₈ aryl, subtituted or not C₂-C₁₀ alkenyl, and subtituted or not C₂-C₁₀ alkynyl;
R³ represents a substituent selected from the group consisting of hydrogen, substituted or not C₁-C₁₀ alkyl, halogen, substituted or not C₃-C₁₀ cycloalkyl, substituted or not C₆-C₁₈ aryl, subtituted or not C₂-C₁₀ alkenyl, and subtituted or not C₂-C₁₀ alkynyl, amino, heterocycle, thiocyanate, thio derivatives, hydroxylamine, amido, sulfonamide, amino sulfone derivatives, ether, thioether, ester, thioester, azido, azo, cyano, nitro, nitrooxy, carbamate, thiocarbamate, sulfinyl derivative, sulfonyl derivatives, acyl derivatives, oxy derivatives, ureido, thioureido, imino, oximino, hydrazino, thioamido, carboxylic, phosphate, phosphonate, carbamoyl phosphate;
with the proviso that when A is 5-fluoroindole and B is bonded at position 3 of the 5-fluoroindole moiety, R¹ is hydrogen and n is between 2 and 10, R³ is not -NR⁴R⁵ wherein R⁴ and R⁵ independently represent a substituent selected from the group consisting of hydrogen, C₁-C₁₀ alkyl, C₃-C₁₀ cycloalkyl, aminoalkyl, or R⁴ and R⁵ taken together with the nitrogen atom to which they are attached to form a four to ten-membered heterocycle, and R² is not independently in each of the n units a substituent selected from the group consisting of hydrogen, C₁-C₁₀ alkyl, halogen, alkoxy, aminoalkyl and alkylamino;
for the treatment or the prophylaxis of neurologic and/or inflammatory diseases or disorders.

In particular, n may be an integer between 2 and 10. Hence, n can be 2, 3, 4, 5, 6, 7, 8, 9 or 10, or a value in the range between any two of the aforementioned values. Preferably, n may be an integer between 2 and 6. Alternatively, n may be an integer between 2 and 5.

The moiety A may be an aromatic N-heterocycle which is substituted or not. Preferably, the aromatic N-heterocycle is selected from the group consisting of indole, imidazole, quinoline, isoquinoline, pyridine, triazine, pyrazine, pyrimidine, pyridazine, isoindole, pyrrole, benzimidazole, purine, pyrazole, benzopyrazole, indazole, quinoxaline, acridine, quinazoline, indolizine, carbazole and cinnoline. The aromatic N-heterocycle may be bicyclic moiety in which the N-heterocycle may be fused with an aryl ring. Preferably, the aromatic N-heterocycle is selected from the group consisting of indole, benzimidazole, quinoline, isoquinoline, isoindole, benzopyrazole. The aromatic N-heterocycle may influence or enhance the inhibition of myeloperoxidase enzyme or LDL oxidation. The affinity to serotonin receptor may also be decreased in function of the aromatic N-heterocycle selected. The aromatic N-heterocycle may be substituted by halogen atom, preferably fluor atom. The aromatic N-heterocycle may also be substituted by -CO₂H or C₁-C₁₀ alkoxy. The aromatic N-heterocycle may also be substituted by one or more as the substituents as defined above.

In a preferred embodiment, the present compound may have the general formula (IIa), (IIb), (IIc), (IId), (IId), (IIe), (IIf), or (IIg) 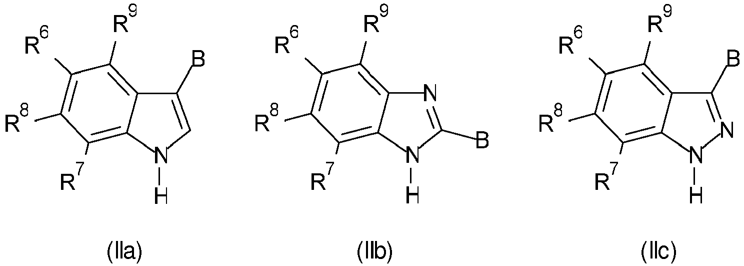 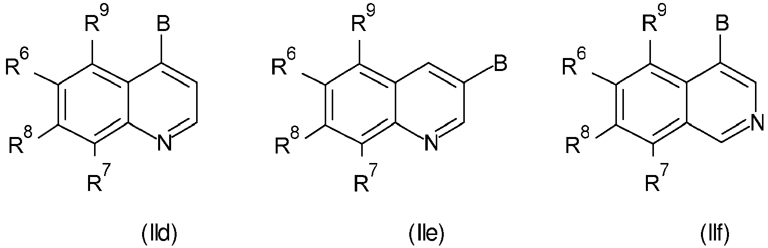 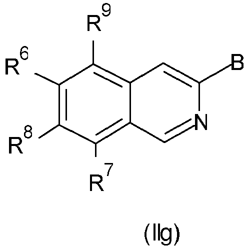 wherein R⁶, R⁷, R⁸ and R⁹ independently represent a substituent selected from the group consisting of hydrogen, F, Cl, Br, CO₂H CN and C₁-C₁₀ alkoxy. B is as defined above.

In a preferred embodiment, A may be a substituted indole moiety of formula (IIa) 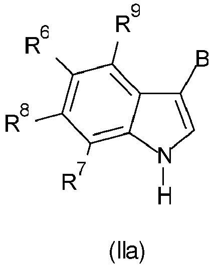 wherein R⁶, R⁷, R⁸ and R⁹ independently represent a substituent selected from the group consisting of hydrogen, F, Cl, Br, CO₂H, CN, C₁-C₁₀ alkoxy; preferably R⁶, R⁷, R⁸ and R⁹ independently represent hydrogen or fluoride. In the general formula (IIa), B is as defined above. The substituents R⁶, R⁷, R⁸ or R⁹ may influence the inhibition properties of the resulting compounds towards MPO or LDL oxidation. The position of the moiety B on the indole ring may also influence the inhibitions properties. The moiety B is preferably bonded to the carbon atom at position 3 of the indole ring as represented on formula (IIa).

Hence, the compounds of the present invention may have general formula (IIIa), (IIIb), (IIIc), (IIId), (IIIe), (IIIf), or (IIIg) 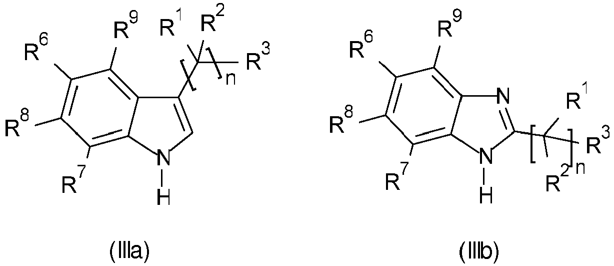 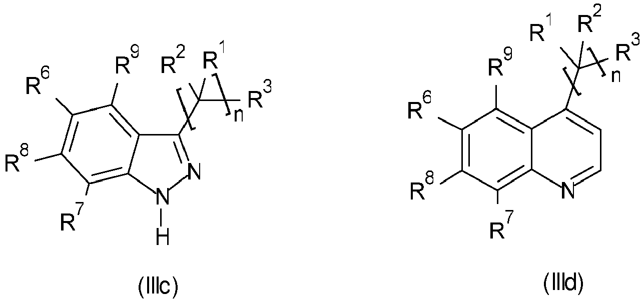 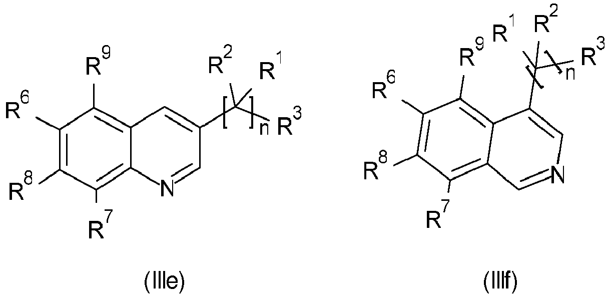 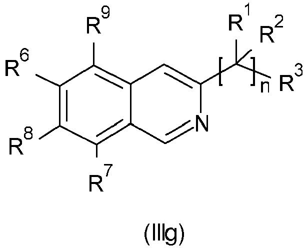 wherein n is an integer between 1 and 10,
R⁶, R⁷, R⁸ and R⁹ independently represent hydrogen, fluoride, chloride, bromide,-CO₂H CN, C₁-C₁₀ alkoxy; preferably R⁶ and R⁷ independently represent hydrogen, F, Br, Cl, CN, CO₂H, C₁-C₁₀ alkoxy and R⁸ and R⁹ are hydrogen; more preferably R⁶ and R⁷ independently represent hydrogen or F and R⁸ and R⁹ are hydrogen;
R¹ and R² represent independently in each of the n units a substituent selected from the group consisting of hydrogen, substituted or not C₁-C₁₀ alkyl, halogen, oxy derivatives, thio derivatives, substituted or not C₃-C₁₀ cycloalkyl, substituted or not C₆-C₁₈ aryl, subtituted or not C₂-C₁₀ alkenyl, and subtituted or not C₂-C₁₀ alkynyl; preferably R¹ and R² represent independently in each of the n units a substituent selected from the group consisting of hydrogen, substituted or not C₁-C₁₀ alkyl;
R³ represents a substituent selected from the group consisting of hydrogen, substituted or not C₁-C₁₀ alkyl, halogen, substituted or not C₃-C₁₀ cycloalkyl, substituted or not C₆-C₁₈ aryl, subtituted or not C₂-C₁₀ alkenyl, and subtituted or not C₂-C₁₀ alkynyl, amino, heterocycle, thiocyanate, thio derivatives, hydroxylamine, amido, sulfonamide, amino sulfone derivatives, ether, thioether, ester, thioester, azido, azo, cyano, nitro, nitrooxy, carbamate, thiocarbamate, sulfinyl derivative, sulfonyl derivatives, acyl derivatives, oxy derivatives, ureido, thioureido, imino, oximino, hydrazino, thioamido, carboxylic, phosphate, phosphonate, carbamoyl phosphate; preferably R³ represents a substituent selected from the group consisting of substituted or not C₁-C₁₀ alkyl, substituted or not C₁-C₁₀ alkoxy, substituted or not C₃-C₁₀ cycloalkyl, substituted or not C₆-C₁₈ aryl, amino, heterocycle, substituted or not thioalkyl, thiocyanate, thio derivatives, hydroxylamine, amido, sulfonamide, amino sulfone derivatives, ether, thioether, ester, thioester, azido, nitro, cyano, nitrooxy, carbamate, thiocarbamate, sulfinyl derivative, alkenyl, alkynyl, acyl derivative, oxy derivatives;
with the proviso that for compounds of formula (IIIa) when R⁶ is fluoride, R¹, R⁷, R⁸ and R⁹ are hydrogen, n is between 2 and 10, then R³ is not -NR⁴R⁵ wherein R⁴ and R⁵ are independently represent a substituent selected from the group consisting of hydrogen, C₁-C₁₀ alkyl, C₃-C₁₀ cycloalkyl, aminoalkyl, or R⁴ and R⁵ taken together with the nitrogen atom to which they are attached to form a four to ten-membered heterocycle, R² is not independently in each of the n units a substituent selected from the group consisting of hydrogen, C₁-C₁₀ alkyl, halogen, alkoxy, aminoalkyl, and alkylamino.

In particular, compounds of the present invention may have general formula (IIIa) 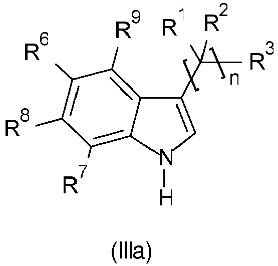 wherein n is an integer between 1 and 10,
R⁶ and R⁷ independently represent hydrogen, F, Br, Cl, CN, CO₂H, C₁-C₁₀ alkoxy; preferably hydrogen or F; R⁸ and R⁹ are hydrogen;
R¹ and R² represent independently in each of the n units a substituent selected from the group consisting of hydrogen, substituted or not C₁-C₁₀ alkyl, halogen, oxy derivative, thio derivative, substituted or not C₃-C₁₀ cycloalkyl, substituted or not C₆-C₁₈ aryl, C₂-C₁₀ alkenyl, C₂-C₁₀ alkynyl;
R³ represents a substituent selected from the group consisting of substituted or not C₁-C₁₀ alkyl, substituted or not C₃-C₁₀ cycloalkyl, substituted or not C₆-C₁₈ aryl, amino, heterocycle, thiocyanate, thio derivatives, hydroxylamine, amido, sulfonamide, amino sulfone derivatives, ether, thioether, ester, thioester, azido, nitro, nitrooxy, carbamate, thiocarbamate, sulfinyl derivative, C₂-C₁₀ alkenyl, C₂-C₁₀ alkynyl, acyl derivative, oxy derivative;
with the proviso that when R⁶ is fluoride, R⁷, R⁸, R⁹ and R¹ are hydrogen and n is between 2 and 10, then R³ is not -NR⁴R⁵ wherein R⁴ and R⁵ are independently represent a substituent selected from the group consisting of hydrogen, C₁-C₁₀ alkyl, C₃-C₁₀ cycloalkyl, aminoalkyl, or R⁴ and R⁵ taken together with the nitrogen atom to which they are attached to form a four to ten-membered heterocycle, R² is not independently in each of the n units a substituent selected from the group consisting of hydrogen, C₁-C₁₀ alkyl, halogen, alkoxy, aminoalkyl, and alkylamino.

Such compounds (IIIa)-(IIIg) are useful as medicine or for the treatment or the prophylaxis of neurologic and/or inflammatory diseases or disorders. Preferably, in compounds of formula (IIIa)-(IIIg) R³ may represent a substituent selected from the group consisting of substituted or not C₁-C₁₀ alkyl, substituted or not C₃-C₁₀ cycloalkyl, substituted or not C₆-C₁₈ aryl, amino, heterocycle, substituted or not thioalkyl, thiocyanate, cyano, hydroxylamine, amido, sulfonamide, amino sulfone derivatives, ether, thioether, thioester, ester, carboxylic, sulfinyl derivative, acyl derivative, oxy derivatives. Alternatively, R³ may represent a substituent selected from the group consisting of substituted or not C₁-C₁₀ alkyl, substituted or not C₁-C₁₀ alkoxy, substituted or not C₃-C₁₀ cycloalkyl, substituted or not C₆-C₁₈ aryl, amino, heterocycle, substituted or not thioalkyl, thiocyanate, cyano, hydroxylamine, amido, amino sulfone derivatives. Alternatively, R³ represents a substituent selected from the group consisting of substituted or not C₁-C₁₀ alkyl, amino, substituted or not thioalkyl, thiocyanate, cyano, amino sulfone derivatives, substituted or not piperidine, hydroxylamine, amido. Alternatively, R³ represents a substituent selected from the group consisting of substituted or not C₁-C₁₀ alkyl, amino, substituted or not thioalkyl, thiocyanate, amino sulfone derivatives, substituted or not piperidine, hydroxylamine, amido. Alternatively, R³ represents a substituent selected from the group consisting of substituted or not C₁-C₁₀ alkyl, cyano, substituted or not thioalkyl, thiocyanate, amino sulfone derivatives, substituted or not piperidine, hydroxylamine, amido; preferably substituted or not thioalkyl, thiocyanate, amino sulfone derivatives, hydroxylamine, amido. Alternatively, R³ may be thioamido. Substituents R¹ and R² may be the same in each n unit. Substituents R¹ and R² may not bear amino functional group.

In a particular embodiment, the present invention relates to compounds, or a pharmaceutically acceptable salt, solvate, hydrate, polymorph, co-crystal thereof, having general formula (IV) 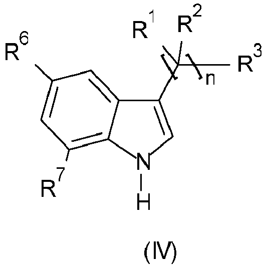 wherein n is an integer between 1 and 10, preferably between 2 and 10, more preferably between 2 and 6;
R⁶ and R⁷ independently represent hydrogen, F, Br, Cl, CN, CO₂H, C₁-C₁₀ alkoxy; preferably hydrogen or F;
R¹ and R² represent independently in each of the n units a substituent selected from the group consisting of hydrogen, substituted or not C₁-C₁₀ alkyl; preferably hydrogen and substituted or not C₁-C₆ alkyl, more preferably hydrogen or non-substituted C₁-C₆ alkyl;
R³ represents a substituent selected from the group consisting of thiocyanate, amino sulfone derivatives, amido, cyano, substituted or not thioalkyl, hydroxylamine, and non-substituted C₁-C₁₀ alkyl; more preferably thiocyanate, amino sulfone derivatives, amido, substituted or not thioalkyl, hydroxylamine, and non-substituted C₁-C₁₀ alkyl; most preferably thiocyanate, amino sulfone derivatives, amido, substituted or not thioalkyl, hydroxylamine; in particular thiocyanate, amino sulfone derivatives, substituted or not thioalkyl, hydroxylamine; more particular thiocyanate, amino sulfone derivatives, substituted or not thioalkyl.
for the treatment or the prophylaxis of neurologic and/or inflammatory diseases or disorders.

In another particular embodiment, the present invention relates to compounds, or a pharmaceutically acceptable salt, solvate, hydrate, polymorph, co-crystal thereof, having general formula (IV) 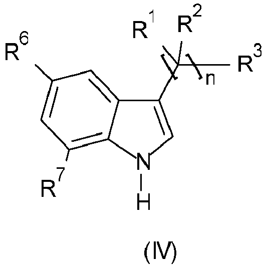 wherein n is an integer between 1 and 10, preferably between 2 and 10, more preferably between 2 and 6;
R⁶ represents hydrogen, Br, Cl, CN, CO₂H, C₁-C₁₀ alkoxy; preferably hydrogen;
R⁷ represents hydrogen, F, Br, Cl, CN, CO₂H, C₁-C₁₀ alkoxy; preferably F;
R¹ and R² represent independently in each of the n units a substituent selected from the group consisting of hydrogen, substituted or not C₁-C₁₀ alkyl; preferably hydrogen and substituted or not C₁-C₆ alkyl, more preferably hydrogen and substituted or not C₁-C₂ alkyl; R³ is amino;
for us as medicine or for the treatment or the prophylaxis of neurologic and/or inflammatory diseases or disorders.

In a more particular embodiment, the present invention relates to compounds having general formula (V) 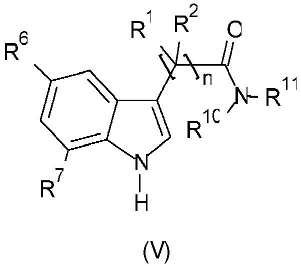 wherein n is an integer between 1 and 10, preferably between 2 and 10, more preferably between 2 and 6;
R⁶ and R⁷ independently represent hydrogen, F, Br, Cl, CO₂H, CN, C₁-C₁₀ alkoxy, preferably hydrogen or F;
R¹ and R² represent independently in each of the n units a substituent selected from the group consisting of hydrogen and substituted or not C₁-C₁₀ alkyl, preferably hydrogen and substituted or not C₁-C₆ alkyl, more preferably hydrogen or non-substituted C₁-C₆ alkyl;
R¹⁰ and R¹¹ independently represent a substituent selected from the group consisting of hydrogen, substituted or not C₁-C₁₀ alkyl, C₂-C₁₀ alkenyl, C₂-C₁₀ alkynyl, C₆-C₁₈ aryl, heterocycle, or R¹⁰ and R¹¹ taken together with the nitrogen atom to which they are attached form a four to ten membered heterocycle;
or a pharmaceutically acceptable salt, solvate, hydrate, polymorph, co-crystal thereof, for the treatment or the prophylaxis of neurologic and/or inflammatory diseases or disorders.

In a preferred embodiment, said compound according to the present invention may have an IC₅₀ value equal or less than 0.1 µM, preferably less than 0.05 µM, and more preferably less than 30 nM against myeloperoxidase enzyme. In particular, said compound according to the present invention may have an IC₅₀ lower than 25 nM against myeloperoxidase enzyme, preferably lower than 10nM, more preferably less than 5 nM. IC₅₀ values obtained are lower than values observed in the prior art. Compounds of the present invention may thus exhibited improved inhibition properties against myeloperoxidase enzyme.

The compounds of the present invention may be in the form of salt, in particular acid addition salt. Suitable salts include those formed with both organic and inorganic acids. Such acid addition salts will normally be pharmaceutically acceptable salts although salts of non-pharmaceutically acceptable acids may be of utility in the preparation and purification of the compound of the present invention. Hence, preferred salts include those formed from hydrochloric, hydrobromic, trifluoroacetic, sulphuric, oxalic, phosphoric, citric, tartaric, lactic, pyruvic, acetic, succinic, fumaric, maleic, methanesulphonic, p-toluenesulfonic, formic, adipic, glycolic, aspartic, malic, oleic, nicotinic, saccharinic and benzenesulphonic acids. The compounds of the present invention may be in the form of hydrate, i.e. it contains water. The compounds of the present invention may be in the form of solvates, i.e. it interacts with solvent. The compounds of the present invention may be in the form of co-crystals, i.e. it crystallizes with a co-crystallizing agent. The compounds of the present invention may exist in one or more crystal structure, all encompassed herein. The compounds of the present invention may be amorphous.

The compounds according to the invention, compounds of formual (I), (IIa-g), (IIIa-g), (IV) or (V), or pharmaceutically acceptable salts thereof, are indicated for use in the treatment or prophylaxis of diseases or disorders in which modulation of the activity of the enzyme myeloperoxidase is beneficial. In particular, linkage of myeloperoxidase activity to disease has been demonstrated in neurologic and/or inflammatory diseases. Therefore, the compound of the present invention is particularly indicated for use in the treatment of neurologic and/or inflammatory disorders or diseases in mammals including human. Such diseases or disorders will be readily apparent to the man skilled in the art.

Disorders or diseases that may be specifically mentioned include multiple sclerosis, Alzheimer's disease, Parkinson's disease, amyotrophic lateral sclerosis and stroke, as well as other inflammatory diseases or disorders such as rheumatic diseases, asthma, chronic obstructive pulmonary disease, cystic fibrosis, idiopathic pulmonary fibrosis, acute respiratory distress syndrome, sinusitis, rhinitis, psoriasis, dermatitis, uveitis, gingivitis, atherosclerosis, inflammatory bowel disease, renal glomerular damage, liver fibrosis, sepsis, proctitis, rheumatoid arthritis, and inflammation associated with reperfusion injury, spinal cord injury and tissue damage/scarring/adhesion/rejection. Lung cancer has also been suggested to be associated with high MPO levels. The compounds are also expected to be useful in the treatment of pain.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the diseases or disorders.

In particular, compounds of the present invention are suitable for the use in the treatment of multiple sclerosis, atherosclerosis, rheumatic diseases, Alzheimer's disease, chronic pulmonar disease, chronic inflammatory syndromes linked to joints or Parkinson's disease. Therefore, the present invention relates to the use of a compound or pharmaceutically acceptable salts thereof according to the present invention for use in the manufacture of a medicament for the treatment of multiple sclerosis, atherosclerosis, rheumatic diseases, Alzheimer's disease, chronic pulmonar disease, chronic inflammatory syndromes linked to joints or Parkinson's disease. Hence, compounds of the present invention may be useful for one or more of the above-mentioned diseases or disorders. In particular, said compounds may be suitable for the treatment of cardiovascular diseases such as atherosclerosis or rheumatic diseases. Said compounds may be used for the manufacture of a medicament for use in the treatment of atherosclerosis or rheumatic diseases.

For the above mentioned therapeutic indications, the dosage administered will, of course, vary with the compound employed, the mode of administration and the treatment desired. However, in general, satisfactory results may be obtained when the compounds are administered at a dosage of the solid form of between 0.1 mg and 2000 mg per day.

In a second aspect of the present invention, a pharmaceutical composition is provided. Said pharmaceutical composition comprises a therapeutically effective amount of a compound according to the present invention, e.g. a compound of formula (I), (IIa-g), (IIIa-g), (IV) or (V), or a pharmaceutically acceptable salt, solvate, hydrate, polymorph, co-crystal thereof, in admixture with a pharmaceutically acceptable adjuvant, diluent or carrier. Said pharmaceutical composition may be used for the treatment or prophylaxis of neurologic and/or inflammatory diseases or disorders. In particular, said neurologic and/or inflammatory diseases or disorders may be selected from the group consisting of multiple sclerosis, atherosclerosis, Alzheimer's disease, chronic pulmonar disease, chronic inflammatory syndromes linked to joints and Parkinson's disease. The term "therapeutically effective amount" refers to dosage that provides the specific pharmacological response for which the drug is administered in a significant number of subjects in need of such treatment. The "therapeutically effective amount" may vary according, for example, the physical condition of the patient, the age of the patient and the severity of the disease. The pharmaceutical composition may comprise less than 80% and more preferably less than 50% of a compound of formula (I), (IIa-g), (IIIa-g), (IV) or (V), or a pharmaceutically acceptable salt, solvate, hydrate, polymorph, co-crystal thereof. Administration of such pharmaceutical composition may be by, but is not limited to, enteral (including oral, sublingual or rectal), intranasal, inhalation, intravenous, topical or other parenteral routes. Conventional procedures for the selection and preparation of suitable pharmaceutical formulations are described in, for example, "Pharmaceuticals - The science of dosage form designs", M.E. Aulton, Churchill Livingstone, 1988.

In another aspect of the present invention in vitro method for inhibiting myeloperoxidase enzyme activity or for inhibiting low density lipoproteins (LDL) oxidation are provided. Said in vitro method for inhibiting myeloperoxidase enzyme activity comprises the step of adding a compound according to the present invention to a medium containing said enzyme, said compound being added in a concentration effective to inhibit the activity of said enzyme. Said medium containing said myeloperoxidase enzyme may be a phosphate buffer. The pH of said medium may be between 7 and 8, preferably between 7.2 and 7.6. Preferably, the pH of said medium may be approximately 7.4. The concentration of the compound according to the present invention effective to inhibit the activity of the enzyme myeloperoxidase may be below 0.1 µM, preferably below 50 nM. A concentration of a compound is considered as effective to inhibit the activity of an enzyme when such concentration inhibit 50% of the enzyme activity. The in vitro method for inhibiting low density lipoproteins (LDL) oxidation may comprise the step of contacting a compound according to the present invention in a medium containing said low density lipoproteins and myeloperoxidase enzyme. Said compound may be added in a concentration effective to inhibit the oxidation of said low density lipoproteins. The concentration effective to inhibit the oxidation of said low density lipoproteins may be lower than 200nM, preferably lower than 100 nM, more preferably lower than 50 nM, most preferably lower than 30 nM.

Compounds according to the present invention of general formula (I), (IIa-g), (IIIa-g), (IV) or (V) for inhibiting myeloperoxidase enzyme activity are also provided. Compounds according to the present invention of formula (I), (Ila-g), (IIIa-g), (IV) or (V) for inhibiting low density lipoproteins oxidation are also provided. In particular, said compounds may further not interact or have limited interaction with serotonine transporter SERT. Pharmaceutical composition according to the present invention may be useful for inhibiting myeloperoxidase enzyme activity or for inhibiting low density lipoproteins oxidation.

Compounds according to the present invention of general formula (I), (IIa-IIg), (IIIa-IIIg), (IV) or (V), or a pharmaceutically acceptable salt, solvate, hydrate, polymorph, co-crystal thereof, may be used as medicine. In particular, compounds having general formula (IV) may be used as medicine 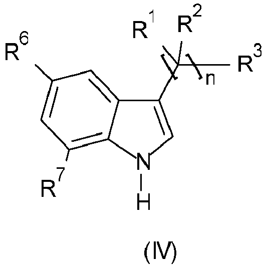 wherein n is an integer between 1 and 10, preferably between 2 and 10, more preferably between 2 and 6;
R⁶ and R⁷ independently represents hydrogen, F, Br, Cl, CO₂H CN, C₁-C₁₀ alkoxy; preferably hydrogen or F;
R¹ and R² represent independently in each of the n units a substituent selected from the group consisting of hydrogen, substituted or not C₁-C₁₀ alkyl; preferably hydrogen and substituted or not C₁-C₆ alkyl, more preferably hydrogen or non-substituted C₁-C₆ alkyl,
R³ represents a substituent selected from the group consisting of thiocyanate, amino sulfone derivatives, amido, cyano, substituted or not thioalkyl, hydroxylamine, and non-substituted C₁-C₁₀ alkyl; more preferably thiocyanate, amino sulfone derivatives, amido, substituted or not thioalkyl, hydroxylamine, and non-substituted C₁-C₁₀ alkyl; most preferably thiocyanate, amino sulfone derivatives, amido, substituted or not thioalkyl, hydroxylamine; in particular thiocyanate, amino sulfone derivatives, substituted or not thioalkyl, hydroxylamine.

In particular, compounds having general formula (V) may also be used as medicine, 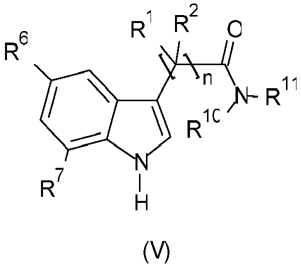 wherein n is an integer between 1 and 10; preferably between 2 and 10, more preferably between 2 and 6;
R⁶ and R⁷ independently represents hydrogen, F, Br, Cl, CO₂H, CN, C₁-C₁₀ alkoxy; preferably hydrogen or F;
R¹ and R² represent independently in each of the n units a substituent selected from the group consisting of hydrogen and substituted or not C₁-C₁₀ alkyl, preferably hydrogen and substituted or not C₁-C₆ alkyl, more preferably hydrogen or non-substituted C₁-C₆ alkyl;
R¹⁰ and R¹¹ independently represent a substituent selected from the group consisting of hydrogen, substituted or not C₁-C₁₀ alkyl, C₂-C₁₀ alkenyl, C₂-C₁₀ alkynyl, C₆-C₁₈ aryl, heterocycle, or R¹⁰ and R¹¹ taken together with the nitrogen atom to which they are attached form a four to ten membered heterocycle.

The present invention also provides a method for the treatment of atherosclerosis or rheumatic diseases characterised in that said method comprises the step of administering an therapeutically effective amount of a compound according to the present invention or a therapeutcially effective amount of a pharmaceutical composition according to the present invention to a patient in need thereof.

### EXAMPLES

The invention is illustrated, but in no way limited, by the following examples.

**Experimental procedure**

¹H- and ¹³C-NMR spectrums were taken on a Bruker Avance 300M Hz spectrometer at 293K. δ are given in ppm relative to TMS and the coupling constants are expressed in Hz. IR analysis was performed with a Shimadzu IR-470 spectrophotometer and the peaks data are given in cm⁻¹. MS were obtained on QTOF 6520 (Agilent, Palo Alto, CA, USA), positive mode, ESI, mode TOF, by diffusion of 0.5 mL/min, by mobile phase HCOOH 0.1 M: CH₃OH (50:50), (VCAP 3500, Source t: 350 °C, fragmentation: 110 v, Shimer: 65 v). All reactions were followed by TLC carried out on Fluka PET-foils silica gel 60 Ⓡ, and compounds were visualized by UV and by spraying Van Urk reagent (0.125 g of p-dimethylaminobenzaldehyde is dissolved in 100 mL of 65% sulfuric acid and 0.1 mL of 5% ferric chloride is added).. Column chromatographies were performed with EchoChrom MP silica 63-200 from MP Biomedicals. Organic solutions were dried over Na₂SO₄ and concentrated with a Buchi rotary evaporator. 4-phenylhydrazine hydrochloride, 7-phenylhydrazine hydrochloride, 4-hydrazinobenzoic acid, dihydrofuran, and dihydropyran were available from Sigma-Aldrich. Microwave-promoted reactions were performed with a Start S microwave oven from Milestone.

### Compounds synthesis

### General Procedures

*General Procedures for the Synthesis of 3-(hydroxyalkyl) indole derivatives (compounds 1, 2, 3, 4, 5, 6 and 27).* A solution of 4-fluorophenylhydrazine hydrochloride, 7-fluorophenylhydrazine hydrochloride or 4-hydrazinobenzoic acid (6.9 mmol) in 4 wt % aqueous H₂SO₄ (10 mL) *(in the case of 4-hydrazinobenzoic H₂SO₄ must be 20 wt* %) and DMA (10 mL) was heated at 100 °C under microwave. To this solution was added the corresponding enol ether, or hydroxyaldehyde (6.9 mmol) dropwise over 2 min. Then the reaction was heated at 175 °C (temperature ramp from room temperature to 100 °C over 8 min, then addition of the reagent, heating to 175 °C over 8 min and maintaining this temperature for 30 min, 1000 W). The reaction mixture was cooled to room temperature and extracted two times with EtOAc (25 mL), and the organic layer was washed with water and evaporated. The crude material was purified by column chromatography (eluent CH₂Cl₂/EtOAc 4:1).

*General Procedures for the Synthesis of Mesylate derivatives (compounds 7-12 and* 28). A solution of alcohol (5.5 mmol) in CH₂Cl₂ (25 mL) was cooled with an ice bath. To this solution TEA (0.44 mL, 5.8 mmol) and methanesulfonyl chloride (0.77 mL, 5.5 mmol) were added (the carboxylic acid-containing alcohol derivatives were dissolved in pyridine and no TEA was added). The solution was stirred at room temperature for 2 h. Then it was washed with H₂O and then dried over Na₂SO₄ and filtered. The solvent was evaporated under reduced pressure. The crude material was purified by flash chromatography (CH₂Cl₂/EtOAc 4:1).

*General Procedures for the Synthesis of Azide derivatives (compounds 13, 14, 15, 16 and 29).* To a solution of sulfonate (3.6 mmol) in DMSO (20 mL), NaN₃ (3.6 mmol) was added, and the solution was stirred at room temperature for 1 h at 100 °C. Then H₂O (30 mL) and CH₂Cl₂ (or EtOAc for compounds **15** and **29**) (30 mL) were added. After decantation, the organic layer was washed with water, dried with Na₂SO₄, filtered, and evaporated under reduced pressure. The residue was purified by column chromatography (CH₂Cl₂).

*General Procedures for the Synthesis of Amine derivatives (compounds 17, 18, 19, 20 and 30.* Palladium on charcoal 10% (50 mg) was added to a solution of azide derivative (4.6 mmol) in EtOH (20 mL). The suspension was stirred under H₂ (4.1 bar) overnight. After filtration on Celite, the solvent was evaporated under reduced pressure. (For the compounds **20** and **30**, after filtration H₂O (30 mL) and EtOAc (30 mL) were added, the aqueous layer was collected and evaporated to give the pure compounds). The residue was dissolved in ether, extracted with 0.1 M HCl, and the resulting solution was washed with ether. A solution of 1 M NaOH was added (pH ≈ 10), and the mixture was extracted with diethylether. The organic layer was washed with water, dried over Na₂SO₄ and evaporated under reduced pressure. A saturated solution of anhydrous oxalic acid in ether was added to the ethereal solution and the resulting solid was filtered, washed with ether, and dried under vacuum.

*General Procedures for the Synthesis of nitrile derivatives (compounds 21 and 22).* To a solution of NaCN (2 g, 41 mmol) in H₂O (10 mL), DMA (10 mL) was added and heated at 100 °C. Then, a solution of the mesylate (7.4 mmol) in DMA (10 mL) was added dropwise. The reaction was stirred at this temperature for 2 h and then cooled to room temperature and extracted with EtOAc. The organic layer was washed with water, dried on Na₂SO₄, and evaporated. The residue was purified by flash chromatography (CH₂Cl₂/EtOAc 4:1).

*General Procedures for the Synthesis of Amide derivatives (compounds 23, 24).* To a solution of nitrile (11 mmol) in *t*-BuOH (40 mL) was added finely powdered KOH (5 g, 89 mmol). The solvent was refluxed for 2 h with stirring. Then a saturated solution of NaCl (50 mL) was added and the resulting mixture was extracted three times with CH₂Cl₂. Flash chromatography was performed (CH₂Cl₂/EtOAc 4:1 then CH₂Cl₂/methanol 1:1). The fractions obtained with the second solvent mixture were evaporated.

*Synthesis of Compound 25.* The amide compound **23** (1.4 g, 6.4 mmol) was dissolved in dioxane (50 mL) and LiAlH₄ (1.0 M solution in dioxane, 25 mL) was added. The suspension was refluxed for 3 h; the reaction was quenched with ice and a 15 wt % KOH aqueous solution (10 mL). The mixture was filtered through Celite and extracted with EtOAc (30 mL). The organic layer was extracted with 0.1 M HCl and, after decantation, the aqueous phase was washed with diethylether. A 1 M KOH solution was added to the acidic layer (pH ≈ 10) and this was extracted with diethylether. The solvent was dried on Na₂SO₄ and evaporated. The residue was dissolved in diethylether, and a saturated solution of anhydrous oxalic acid in diethylether was added drops wise. The precipitate was filtered, washed with ether, and dried at 40 °C under reduced pressure.

*Synthesis of Compound 26.* A 1 M DIBAL-H solution in CH₂Cl₂ (7.8 mL, 7.38 mmol) was added slowly to a solution of the lactone (6.6 mmol) in dry CH₂Cl₂ at -78 °C. After stirring at the same temperature for 1 h, methanol (8.9 mL) was added dropwise and the mixture poured into 0.5 M HCl (200 mL). After stirring for additional 1 h at room temperature, the organic layer was separated. The aqueous layer was extracted with CH₂Cl₂ (3 ×20 mL), and the combined organic layers were dried with Na₂SO₄ and evaporated. The residue was purified by flash chromatography (first CH₂Cl₂, then CH₂Cl₂:EtOAc (60:40)). The second fraction was evaporated to give the desired product.

*General Procedure for the Synthesis of Compounds 32-36 and 41-46.* A solution of mesylate **7**, **8**, **11** or **12** (3.6 mmol) in dioxane (5 mL) was added slowly through an addition funnel to a refluxing solution of the amine or thiol (0.26 mol) in dioxane (15 mL) at 100 °C (for some compounds see special conditions summarized in table 1). After the addition was completed, the reaction medium was stirred at this temperature for 4 h. After cooling, the mixture was treated with water (20 mL) and extracted with EtOAc (30 mL). The organic layer was dried over Na₂SO₄ and evaporated to dryness to afford a crude product. The residue was dissolved in 0.1 M HCl. This solution was washed with diethylether and rendered alkaline (pH ≈ 10) with a solution of 1 M NaOH. The mixture was extracted with ether. The organic layer was washed with water, dried over Na₂SO₄, and evaporated to afford the pure compounds. Some compounds were transformed (**31**, **32**, **42**, **43** and **44**) into oxalic salts, by adding saturated solution of anhydrous oxalic acid in ether to their ethereal solution, and then the resulting solid was filtered, washed with ether, and dried under vacuum.

### 7-Fluoro-3-(3-hydroxypropyl)-1H-indole (2).

A solution of 2-fluorophenylhydrazine hydrochloride (1.1 g, 6.9 mmol) in 4% aqueous H₂SO₄ (10 mL) and DMAc (10 mL) was heated at 100 °C. To this solution was added dihydropyran (630 µL, 6.9 mmol) dropwise over 2 min. The reaction was heated at 100 °C for 2 h, then the reaction mixture was cooled to room temperature, and extracted with EtOAc. The organic layer was washed with water, dried over anhydrous Na₂SO₄, filtered and evaporated. The crude material was purified by flash chromatography using CH₂Cl₂: EtOAc (80:20) to afford a yellow oil (1.25 g, 95% yield). ¹H NMR (CDCl₃) δ 8.25 (br s, 1H), 7.27 (d, 1H, *J* = 7.8 Hz), 6.92 (dt, 1H, *J*=7.8Hz, 4.8Hz), 6.83 (d, 1H, *J*= 0.6 Hz),6.81 (dd, 1H, *J*=11.1Hz, 7.5Hz), 3.62 (t, 2H, *J* = 6.3 Hz,), 2.75 (t, 2H, *J* = 7.5 Hz), 1.89 (m, 2H); ¹³C NMR (CDCl₃) δ 149.6 (d, *J* = 242 Hz), 131.1 (d, *J* = 5 Hz), 124.5 (d, *J* = 13 Hz), 121.9 (s), 119.2 (d, *J* = 6 Hz), 116.7 (d, *J* = 2 Hz), 114.5 (d, *J* = 3 Hz), 106.6 (d, *J* = 16 Hz), 62.4 (s), 32.8 (s), 21.3 (s); IR (film) 3395, 2920, 1711, 1620, 1576, 1495, 1443, 1336, 1250, 1220, 1182, 1040, 963, 923, 861, 780, 747 cm⁻¹.

### 3- (3-Hydroxypropyl) 1H-indole-5-carboxylic acid (3)

A solution of 4-hydrazinobenzoic acid (1 g, 6.6 mmol) in 20% aqueous H₂SO₄ (10 mL) and DMAc (10 mL) was heated at 100 °C. To this solution was added dihydropyran (1000 µL, 11 mmol) dropwise over 2 minutes. Then the reaction was refluxed at 100 °C for 4 hours. The reaction mixture was cooled to room temperature and extracted with EtOAc and the organic layer was washed with water and evaporated. The crude material was purified by flash chromatography by using CH₂Cl₂: EtOAc (80:20) (solution 1), then by CH₂Cl₂: EtOAc (50:50) (solution 2). The solution 2 was evaporated to afford a white solid (600 mg, 42 % yield). 1H NMR (DMSO-*d₆*) δ 12.37 (br s, 1H, COOH), 11.14 (br s, 1H), 8.20 (s, 1H), 7.70 (dd, 1H, *J* = 8.4, 1.5 Hz), 7.38 (d, 1H, *J* = 8.4 Hz), 7.21 (d, 1H, *J* = 1.5 Hz), 3.50 (t, 2H, *J* = 6.3 Hz,), 2.77 (t, 2H, *J* = 7.8 Hz), 1.83 (m, 2H); 13C NMR (DMSO-*d₆*) δ 168.5 (COOH), 138.8 (s), 126.7 (s), 123.7 (C-3), 122.1 (s), 121.1 (s), 120.6 (s), 116.0 (s), 111.0 (s), 60.4 (s), 33.3 (s), 20.9 (s); IR (KBr) 3250, 2930, 2625, 1660, 1600, 1518, 1413, 1302, 1255, 1179, 1116, 1084, 844, 770 cm⁻¹ .

### 3-(3,4-Dihydroxybutyl)-5-fluoro-1H-indole(4)

The title compound has been synthesized according to the procedure for the synthesis of 2 but with using (tetrahydropyran-2-yl)methanol to afford yellowish solid (200 mg, 23 % yield). ¹H NMR (CDCl₃) δ 8.49 (br s, 1H), 7.23 (dd, 1H, *J*= 8.7, 4.5 Hz), 7.25 (d, 1H, *J* = 2.1 Hz), 7.18 (dd, 1H, *J*= 9.7, 2.7 Hz), 6.89 (dt, 1H, *J* = 9, 2.1 Hz), 3.81 (dq, 1H, *J*=6.8, 3.2 Hz), 3.68(dd, 1H, *J*=11.2, 3.2 Hz), 3.50 (dd, 1H, *J*=11.2, 7.2 Hz), 2.94 (sept, 1H, *J*=7.2 Hz), 2.90 (sept, 1H, *J*=7.2 Hz), 1.88 (m, 2H).

### 5-Fluoro-3-(2-hydroxyethyl)-1H-indole (5)

The title compound has been synthesized according to the procedure for the synthesis of 2 starting from 4-fluorophenylhydrazine hydrochloride to afford yellow oil (1.1 g, 95% yield). ¹H NMR (CDCl₃) δ 8.33 (br s, 1H), 7.30 (m, 2H), 7.11 (d, *J*= 2.1 Hz, 1H), 6.98 (dt, 1H, *J*= 9.2, 2.4 Hz), 3.93 (t, 2H, *J*= 6.4 Hz), 3.01 (t, 2H, *J*= 6.6 Hz), 2.03 (br s, 1H, OH); ¹³C NMR (CDCl₃) δ 157.9 (d, J= 232 Hz), 133.1 (s), 127.8 (d, *J=* 10 Hz), 124.5 (s), 122.5 (d, *J=* 5 Hz), 111.9 (d, *J=* 10 Hz), 110.5 (d, *J=* 25 Hz), 103.7 (d, *J=* 24 Hz), 62.6 (s), 28.8 (s); IR (film) 3423, 2922, 2880, 1581, 1485, 1230, 1171, 1055 cm⁻¹.

### 5-Fluoro-3-(3 hydroxypropyl)-1H-indole (6)

Synthesized using the same procedure as for the synthesis of compound **5** to afford (yellow oil, 1.25 g, 95% yield). ¹H NMR (CDCl₃) δ 8.22 (br s, 1H), 7.30 (m, 2H), 6.99 (d, *J=* 2.1 Hz, 1H), 6.82 (dt, 1H, *J=* 9.2, 2.4 Hz), 3.72 (t, 2H, *J*= 6.4 Hz), 2.80 (t, *J*= 7.6, 0.8 Hz, 2H), 1.90 (m, 2H, H-2'); IR (film) 3423, 2922, 2880, 1581, 1485, 1230, 1171, 1055 cm⁻¹.

### 6-Hydroxyhexanal (7)

A solution of 1 M diisobutylaluminium hydride (7.8 mL, 7.38 mmol) was added slowly to a solution of ε-caprolactone (0.75 g, 6.6 mmol) in dry CH₂Cl₂ at -78 °C. After stirring at the same temperature for 1 h, MeOH (8.9 mL) was added dropwise and the mixture poured into 0.5 M HCl (200 mL). After stirring for additional 1 h to room temperature, the organic layer was separated. The aqueous layer was extracted, dried and evaporated. The residue purified by flash chromatography with two steps. The first was by CH₂Cl₂, and the second was by CH₂Cl₂: EtOAc( 60:40). The second solution was evaporated to give 0.66 g of **16** (87 %) colorless liquid. ¹H NMR (CDCl₃) δ 9.71 (d, *J =* 1.7 Hz, 1H, CHO), 3.69 (t, *J* =6.53 Hz, 2H), 3.41 (bs, 1H, OH), 2.62 (m, 2H), 1.51 (m, 2H), 1.30 (m, 4H); ¹³C NMR (CDCl₃) δ 202.4 (CHO), 62.7 (s), 43.9 (s), 32.0 (s), 22.5 (s), 21.6 (s); IR (film) 3385, 2915, 1719, 1458, 1395, 1370, 1121, 1047 cm⁻¹.

### 3-(4-Hydroxybutyl)-1H-indole-5-carboxylic acid (10)

The title compound has been synthesized according to the procedure for the synthesis of **3** by using 6-Hydroxyhexanal to afford yellowish oil (550 mg, 41% yield). ¹H NMR (DMSO-*d*₆) δ 11.12 (br s, 1H), 8.18 (s, 1H), 7.69 (dd, 1H, *J* = 8.4, 1.5 Hz), 7.37 (d, 1H, *J* = 8.7 Hz), 7.21 (d, 1H, *J* = 2.1 Hz), 3.43 (t, 2H, *J* = 6.3 Hz), 2.71 (t, 2H, *J* = 7.8 Hz), 1.67 (m, 2H), 1.52 (m, 2H); ¹³C NMR (DMSO-*d*₆) δ 168.5 (COOH), 138.8 (s), 126.8 (s), 123.8 (s), 122.1 (s), 121.0 (s), 120.7 (s), 116.3 (s), 111.0 (s), 60.6 (s), 32.5 (s), 26.5 (s), 24.4 (s); IR (KBr) 3250, 2930, 2625, 1660, 1600, 1518, 1413, 1302, 1255, 1179, 1116, 1084, 844, 770 cm⁻¹.

### 7-Fluoro-3- (3-hydroxypropyl)-1H-indole methanesulfonate (12)

A solution of **2** (1 g, 5.2 mmol) in CH₂Cl₂ (25 mL) was cooled with an ice bath. To this solution TEA (0.7 mL, 3.2 mmol) and methanesulfonyl chloride (0.4 mL, 5.5 mmol) were added. The solution was stirred at room temperature for 2 h. Then, it was washed with a saturated solution of NaHCO₃ and H₂O, dried over Na₂SO₄ and filtered. The solvent was evaporated under reduced pressure. The crude material was purified by flash chromatography CH2Cl2:EtOAc (4:1) to afford compound 7 as a brown oil (1.3 g, 90% yield). ¹H NMR (CDCl₃) δ (br s, 1H), 7.34 (d, 1H, *J* = 7.8 Hz), 7.07 (d, 1H, *J*= 2.1Hz), 7.03 (dt, 1H, *J*=7.8Hz, 4.8Hz), 6.91 (dd, 1H, *J=* 11.1Hz, 7.5Hz), 4.25 (t, 2H, *J*= 6.3 Hz), 2.98 (s, 3H), 2.88 (t, 2H, J = 7.5 H), 2.13 (m, 2H); ¹³C NMR (CDCl₃) δ 149.6 (d, *J*= 242 Hz), 131.1 (d, *J*= 5 Hz), 124.5 (d, *J*= 16 Hz), 122.5 (s), 119.5 (d, *J*= 6 Hz), 115(d, *J* = 2 Hz), 114.3 (d, *J*=3 Hz), 106.8 (d, *J* = 16 Hz), 69.3 (s), 37.2 (SCH₃) 29.3 (s), 20.8 (s); IR (film) 3395, 2920, 1711, 1620, 1576, 1495, 1443, 1336, 1250, 1220, 1182, 1040, 963, 923, 861, 780, 747 cm⁻¹.

### 3- (3-Methanesulfonatopropyl)-1H-indole-5-carboxylic acid (13)

A solution of 3 (1 g, 4.6 mmol) in pyridine (10 mL) was cooled in ice bath. To this solution methanesulfonyl chloride (0.77 mL, 10 mmol) was added. The solution was stirred at room temperature for 4 h. Then 0.5 M H₂SO₄ (50 mL) was added (pH ≈ 2) and the solution was extracted with EtOAc (20 mL) two times, washed with water, dried by Na₂SO₄ and evaporated. The crude product was purified by column chromatography CH₂Cl₂:EtOAc (80:20) to afford a brown oil (800 mg, 61% yield) ¹H NMR (CDCl₃) δ 8.42 (s, 1H), 8.29 (br s, 1H, NH), 7.95 (dd, 1H, *J* = 8.4, 1.5 Hz), 7.37 (d, 1H, 1H, *J* ₌ 8.7 Hz), 7.10 (s, 1H), 4.26 (t, 2H, *J* = 6.3 Hz), 3.00 (s, 3H), 2.94 (t, 2H, *J* = 7.5 Hz), 2.15 (m, 2H); ¹³C NMR (CDCl₃) δ 172.5 (COOH), 139.5 (s), 126.9 (s), 124.0 (s), 123.3 (s), 122.6 (s), 120.5 (s), 116.1 (s), 111.0 (s), 69.1 (s), 37.4 (SCH₃), 29.4 (s), 20.7 (s); IR (film) 3295, 2930, 2530, 1755, 1682, 1600, 1519, 1420, 1337, 1259, 1218, 1180, 1028, 969, 837, 760, 523 cm-1.

### 3-(3,4-Dihydroxybutyl)-5-fluoro-1H-indole dimethanesulfonate (14)

The title compound has been synthesized according to the procedure for the synthesis of **12** but by adding 4 equivalents of TEA and methanesulfonyl chloride to afford yellowish solid (200 mg, 23 % yield). ¹H NMR (CDCl₃) δ 8.49 (br s, 1H), 7.26 (dd, 1H, *J=* 9, 4.5 Hz), 7.18 (dd, 1H, *J*= 9.6, 2.4 Hz), 7.07(d, 1H, *J=* 2.1 Hz), 6.90 (dt, 1H, *J*= 9.0, 2.1 H), 4.91 (m, 1H), 4.36 (dd, 1H, *J=* 11.7, 2.7 Hz), 4.23 (dd, 1H, *J=* 11.4, 6.0 Hz), 3.05 (s, 3H, SCH₃), 2.99 (s, 3H, SCH₃), 2.81 (m, 2H), 2.01 (m,2H); ¹³C NMR (CDCl₃) δ 157.5 (d, *J*= 229 Hz), 133 (s), 127 (d, *J*= 10 Hz), 124.2 (s), 113.7 (d, *J*= 5 Hz), 112.1 (d, *J*= 10 Hz), 110.3 (d, *J*= 26 Hz), 103.4 (d, *J*= 23 Hz), 78.7 (s), 69.9 (s), 38.7(SCH3), 37.5(SCH3), 31.2 (s), 20 (s).

### 5-Fluoro-3-(2-hydroxyethyl)-1H-indole methanesulfonate (15)

Synthesized using the same procedure as for the synthesis of compound 12 to afford a brown oil (1.3 g, 90% yield). ¹H NMR (CDCl₃) δ 8.27 (br s, 1H), 7.27 (dd, 1H, *J=* 8.7, 4.5 Hz), 7.24 (dd, 1H, *J=* 9.7, 2.7 Hz), 7.13 (s, 1H), 6.92 (dt, 1H, *J=* 9.2, 2.4 Hz), 4.44 (t, 2H, *J=* 6.9 Hz), 3.16 (t, 2H, *J=* 6.9 Hz), 2.87 (s, 3H, -CH₃); ¹³C NMR (CDCl₃) δ 157.9 (d, *J=* 232 Hz), 132.4 (ds), 127.2 (d, *J=* 10 Hz), 124.2 (s), 111.7 (d, *J=* 10 Hz), 110.5 (d, *J=* 25 Hz), 110.2 (d, *J=* 5 Hz), 103.7 (d, *J=* 24 Hz), 69.4 (s), 37.1 (CH3), 25.2 (s); IR (film) 3423, 2880, 1581, 1485, 1350, 1230, 1171, 1055 cm⁻¹.

### 5-Fluoro-3-(3-hydroxypropyl)-1H-indole methanesulfonate (16)

The title compound has been synthesized according to the procedure for the synthesis of **12** to afford brown oil (1.26g, 90% yield). ¹H NMR (CDCl₃) δ 8.15 (br s, 1H), 7.25 (dd, 1 H, J= 9.0, 4.2 Hz), 7.21 (dd, 1H, *J=* 7.6, 0.9 Hz), 7.02 (d, 1H, *J=* 1.1 Hz), 6.92 (dt, 1H, *J=* 9.2, 2.4 Hz), 4.22 (t, 2H, *J=* 7.5 Hz), 2.96 (s, H, -CH3), 2.82 (t, 2H, *J=* 7.8 Hz), 2.09 (m, 2H); IR(film) 3423, 2880, 1581, 1485, 1350, 1230, 1171, 1055 cm⁻¹.

### 3- (3-Azidopropyl)-7-fluoro-1H-indole (21)

To a solution of **12** (1 g, 3.7 mmol) in DMSO (20 mL), NaN₃ (700mg, 11.7 mmol) was added and the solution was stirred at room temperature for 4 h. Then H₂O (30 mL) and toluene (30 mL) were added. After decantation, the organic layer was washed with water, dried with Na₂SO₄, filtered and evaporated under reduced pressure. The residue was purified by column chromatography (CH₂Cl₂) to afford yellow oil (588 mg, 80% yield). ¹H NMR (CDCl₃) δ 8.78 (br s, 1H), 7.32 (dd, 1H, *J* = 7.8, 0.6 Hz), 6.98 (dt, 1H, *J=* 7.8Hz, 4.8Hz), 6.95 (d, 1H, *J=* 1.8 Hz), 6.87 (dd, 1H, *J=* 11.4Hz, 7.5Hz), 3.27 (t, 2H, *J =* 6.6 Hz) 2.80 (t, 2H, *J =* 7.5 Hz), 1.94 (m, 2H); ¹³C NMR (CDCl₃) δ 149.6 (d, *J=* 242 Hz), 131.0 (d, *J =* 5 Hz), 124.5 (d, *J =* 13 Hz), 122.2 (s), 119.1 (d, *J =* 6 Hz), 115.4 (s), 114.3 (d, *J* = 3 Hz), 106.4 (d, *J =* 16 Hz), 50.6 (s), 29.0 (s), 21.9 (s); IR (film) 3410, 2920, 2095, 1713, 1633, 1575, 1497, 1442, 1364, 1218, 1180, 1083, 1041, 1010, 960, 781, 742, 669, 336, 502 cm⁻¹.

### 3-(3-Azidopropyl)-1H-indole-5-carboxylic acid (22)

To a solution of **13** (1 g, 3.4 mmol) in DMSO, NaN₃ (700 mg, 12 mmol) was added. The solution was stirred at 100 °C for 4 h. Then the reaction mixture was extracted with EtOAc and washed three times with water, dried with Na₂SO₄, evaporated and purified by flash chromatography CH₂Cl₂: EtOAc (8:2) to give 800 mg of 39 yellow oil (96 % yield) ¹H NMR (CDCl₃) δ 10.19 (br s, 1H, NH), 7.95 (s, 1H), 7.45 (dd, 1H, *J =* 8.4, 1.5 Hz), 7.01 (d, 1H, *J =* 8.7 Hz), 6.71 (d, 1H, *J =* 2.1 Hz), 2.96 (t, 2H, *J =* 6.9 Hz), 2.49 (t, 2H, *J =* 7.2 Hz), 1.61 (m, 2H); ¹³C NMR (CDCl₃) δ 168.7 (COOH), 138.3 (s), 125.9 (s), 122.6 (s), 121.9 (s), 120.7 (s), 120.2 (s), 114.3 (s), 110.1 (s), 49.8 (s), 28.4 (s), 21.1 (s); IR (film) 3410, 2930, 2700, 2170, 1665, 1574, 1427, 1263, 762 cm⁻¹.

### 3-(3,4-Diazidobutyl)-5-fluoro-1H-indole (23)

The title compound has been synthesized according to the procedure for the synthesis of 14 but by adding 4 equivalents of NaN₃ to afford yellowish solid (200 mg, 23 % yield). ¹H NMR (CDCl₃) δ 8.19 (br s, 1H), 7.26 (dd, 1H, *J=* 9.3, 4.2 Hz), 7.20 (dd, 1H, *J=* 9.6, 2.4 Hz), 7.05 (d, 1H, *J=* 2.4 Hz), 6.94 (dt, 1H, *J=* 9.0, 2.4 Hz), 3.39 (m, 3H), 2.83 (m, 2H), 1.87 (m, 2H); ¹³C NMR (CDCl₃) δ 157.5 (d, *J=* 229 Hz), 133 (s), 127.8 (d, *J=* 10 Hz), 123.9 (s), 115.0 (d, *J=* 5 Hz), 112.3 (d, *J=* 10 Hz), 110.9 (d, *J=* 26 Hz), 103.9 (d, *J=* 23 Hz), , 61.7 (s), 55.4 (s), 32.4 (s), 21.9(s).

### 3-(4-Azidobutyl)-1H-indole-5-carboxylic acid (25)

The Mesylate derivative 3- (4-Methanesulfonatobutyl)-*1H*-indole-5-carboxylic acid **19** has been synthesized according to the procedure for the synthesis of **13** to afford yellowish oil (800 mg, 61% yield); this compound was used directly without further purification. Then 3-(4-Azidobutyl)-*1H*-indole-5-carboxylic acid **25** has been synthesized according to the procedure for the synthesis of **21** to afford yellowish oil (800 mg, 96 % yield) ¹H NMR (CDCl₃) δ 8.51 (br s, 1H, NH), 8.44 (s, 1H), 7.96 (dd, 1H, *J* = 8.4, 1.5 Hz), 7.83 (d, 1H, *J* = 8.7 Hz), 6.06 (d, 1H, *J* = 2.1 Hz), 3.31 (t, 2H, *J* = 6.0 Hz), 2.83 (t, 2H, *J = 9.0* Hz), 1.79 (m, 2H), 1.72 (m, 2H); ¹³C NMR (CDCl₃) δ 171.9 (COOH), 139.5 (s), 127.3 (s), 123.9 (s), 122.8 (2 s), 120.2 (s), 117.8 (s), 111.1 (s), 51.5 (s), 28.9 (s), 27.4 (s), 24.7 (s); IR (film) 3245, 2930, 2090, 1693, 1574, 1427, 1263, 1012 cm⁻¹.

### 3-(3-Aminopropyl)-7-fluoro-1H-indole oxalate (27)

Palladium on charcoal 10% (50 mg) was added to a solution of **21** (1 g, 4.9 mmol) in ethanol (20 mL). The suspension was stirred under H₂ (60 psi) overnight in a Parr hydrogenation apparatus. After filtration on celite, the solvent was evaporated under reduced pressure. The product was dissolved in ether (20 mL), washed with water, and dried over Na₂SO₄. To this solution, a saturated solution of anhydrous oxalic acid in ether was added and the resulting solid was filtered, washed with ether, and dried to afford yellowish crystals (0.9 g, 70% yield). ¹H NMR (DMSO-*d₆*) δ 11.35 (br s, 1H), 7.32 (d, 1H, *J =* 7.8Hz), 7.21 (s, 1H), 6.92 (dt, 1H, *J=* 7.5 Hz, 5.1 Hz) 6.87 (dd, 1H, *J=* 11.4Hz, 7.5Hz, 0.3Hz), 2.75 (m, 4H), 1.90 (m, 2H); ¹³C NMR (DMSO-*d₆*) δ 166.6 (COO), 149.6 (d, *J* = 242 Hz), 131.0 (d, *J =* 5 Hz), 123.9 (d, *J =* 13 Hz), 123.6 (s), 118.3 (d, *J* = 6 Hz), 114.5 (d, *J= 2* Hz),114.5 (d, *J =* 2 Hz), 105.6 (d, *J =* 17 Hz), 40.1 (s), 28.1 (s), 21.6 (s); IR (film) 3395, 3050, 2915, 1710, 1605, 1575, 1511, 1441, 1217, 721, 123 cm⁻¹; HRMS (ESI) calculated for C₁₁H₁₄N₂F (M+H): 193.1136, found: 193.1198, error 1.30 ppm.

### 3-(2-Aminoethyl)-7-fluoro-1H-indole oxalate (26)

The title compound has been synthesized according to the procedure for the synthesis of **27** starting from azide derivative which was obtained according to the procedure for the synthesis of **21** to afford yellowish crystals (0.9 g, 70% yield). ¹H NMR (DMSO-*d₆*) δ 11.48 (br s, 1H), 7.37 (d, 1H, *J =* 7.8Hz), 7.30 (s, 1H), 6.95 (dt, 1H, *J=* 7.5 Hz, 4.8Hz), 6.90 (dd, 1H, *J=* 11.1 Hz, 0.3Hz), 3.05 (m, 4H); ¹³C NMR (DMSO-*d₆*) δ 164.5 (COO), 148.6 (d, *J =* 242 Hz), 130.8 (d, *J =* 5 Hz), 124.5 (s), 123.9 (d, *J =* 13 Hz), 118.7 (d, *J =* 6 Hz), 114.2 (d, *J =* 3 Hz), 110.7 (d, *J =* 2 Hz), 105.8 (d, *J =* 17 Hz), 39.2 (s), 23.0 (s); IR (film) 3395, 3050, 2915, 1710, 1605, 1575, 1511, 1441, 1217, 721, 123 cm⁻¹; HRMS (ESI) calculated for C₁₀H₁₁N₂F (M⁺): 178.0904, found: 178,0906, error: 1.12 ppm.

### 3- (3-Aminopropyl)-1H-indole-5-carboxylic acid (28)

Palladium on charcoal 10% (50 mg) was added to a solution of **22** (1 g, 4.1 mmol) in ethanol (20 mL). The suspension was stirred under H₂ (60 psi) overnight in a Parr hydrogenation apparatus. After filtration on celite, the solvent was evaporated under reduced pressure. The product was dissolved in EtOAc (20 mL) and water (20 mL). The aqueous layer was washed two times with EtOAc and evaporated to afford 200 mg of compound **28** white solid (23 % yield) ¹H NMR (DMSO-*d₆*) δ 8.08 (s, 1H), 7.63 (dd, 1H, J = 8.4, 1.5 Hz), 7.26 (d, 1H, J = 8.7 Hz), 7.09 (s, 1H), 2.84 (t, 2H, J = 7.5 Hz), 2.75 (t, 2H, J = 7.5 Hz), 1.91 (m, 2H); ¹³C NMR (DMSO-*d₆*) δ 174.4 (COOH), 138.5 (s), 129.2 (s), 127.3 (s), 124.2 (s), 124.0 (s), 121.3 (s), 115.5 (s), 111.5 (s), 39.9 (s), 28.7 (s), 22.6 (s); IR (KBr) 3285, 3105, 2985, 2840, 2105, 1614, 1540, 1513 1362, 1248, 1214, 1125, 1091, 1125, 780, 644, 534 cm⁻¹; HRMS (ESI) calculated for C₁₂H₁₄N₂O₂ (M⁺): 218,105, found: 218,1055, error: 2.29 ppm.

### 3- (4-Aminobutyl)-7-fluoro-1H-indole oxalate (25)

The azido derivative **24** (1.4 g, 6.4 mmol), which was synthesized according to the procedure of compound **21**, was dissolved in dioxane (50 mL) and LiAlH₄ (1.0 M solution in dioxane, 25 mL, 25 mmol) was added. The suspension was refluxed for 2 h and the reaction was quenched with ice and 15% KOH (10 mL). The mixture was filtered through celite and extracted with EtOAc (10 mL). The organic layer was extracted with 0.1 M HCl (10 mL) and, after decantation; the aqueous phase was washed by EtOAc. To the acidic layer, 1 M KOH was added (pH ≈ 10) and this was extracted with diethylether. The solvent was dried on Na₂SO₄ and evaporated. The residue was dissolved in diethylether and a saturated solution of anhydrous oxalic acid in diethylether was added dropwise. The precipitate was filtered, washed with ether and dried at 40 °C under reduced pressure to give a white solid (0.6 g, 26% yield). ¹H NMR (DMSO-*d₆*) δ 11.31 (br s, 1H), 7.34 (d, 1H, *J=* 7.8 Hz), 7.19 (d, *J=* 1.8 Hz, 1H), 6.93 (dt, 1H, *J=* 7.8Hz, 5.1 Hz), 6.89 (dd, *J=* 10.8Hz, 7.5Hz), 2.8 (t, 2H, *J =* 7.5 Hz), 2.67 (t, 2H, *J =* 6.9 Hz), 1.62 (m, 4H); ¹³C NMR (DMSO-*d₆*) δ 164.6 (COO), 149.6 (d, *J* = 242 Hz), 131.1 (d, *J =* 5 Hz), 123.9 (d, *J =* 13 Hz), 123.5 (s), 118.2 (d, *J =* 6 Hz), 115.1 (s), 114.5 (d, *J =* 3 Hz), 105.5 (d, *J=* 17 Hz), 38.6 (s), 26.8 (s), 26.6 (s), 24.1 (s); IR (film) 3395, 3050, 2915, 1710, 1605, 1575, 1511, 1441, 1217, 721, 123 cm⁻¹; HRMS (ESI) calculated for C₁₂H₁₅N₂F (M⁺): 206.1219, found: 206.1219, error: 0.00 ppm

### 3- (4-Aminobutyl)-1H-indole-5-carboxylic acid (31)

The title compound has been synthesized according to the procedure for the synthesis of **27** to afford yellowish solid (200 mg, 23 % yield). ¹H NMR (D₂O) δ 8.06 (s, 1H), 7.62 (dd, 1H, *J* = 8.4, 1.5 Hz), 7.30 (d, 1H, *J* = 8.7 Hz), 6.92 (s, 1H), 2.74 (m, 2H), 2.52 (m, 2H), 1.46 (m, 4H); ¹³C NMR (D₂O) δ 176.9 (COOH), 138.3 (s), 127.1 (s), 126.7 (s), 123.9 (s), 123.1 (s), 120.8 (s), 116.3 (s), 111.3 (s), 39.9 (s), 26.9 (s), 26.7 (s), 23.9 (s); IR (KBr) 3285, 3105, 2985, 2840, 2105, 1614, 1540, 1513 1362, 1248, 1214, 1125, 1091, 1125, 780, 644, 534 cm⁻¹; HRMS (ESI) calculated for C₁₃H₁₆N₂O₂ (M⁺): 232.12091, found: 232.12115, error: 1.03 ppm.

### 3-(2-(2-Aminoethylthio)ethyl)-5-fluoro-1H-indole oxalate (32)

A solution of mesylate derivative **15** (1 g, 3.6 mmol) in dioxane (5 mL) was added slowly through an addition funnel to a refluxing solution of cysteamine hydrochloride and finely powdered KOH in methanol 15 ml at 100 °C. After the addition was completed, the reaction medium was stirred at this temperature for 4 h. After cooling, the mixture was treated with KOH 1 M (20 mL) and extracted with EtOAc (30 mL). The organic layer was washed three times by KOH 1 M and dried over Na₂SO₄ and evaporated to dryness to afford a crude product. The residue was dissolved in 0.1 M HCl. This solution was washed with diethylether and rendered alkaline (pH ≈ 10) with a solution of 1 M NaOH. The mixture was extracted with EtOAc. The organic layer was washed with water, dried over Na₂SO₄ and evaporated and dissolved in ether, to this solution a saturated solution of oxalic acid in ether was added and the pracipitates were collected and dried at 45 °C to give yellow solid. ¹H NMR (DMSO-*d*₆) δ 11.03 (br s, 1H),7.33 (dd, 1H, 9, 4.5 Hz) 7.28 (m, 2H), 6.89 (dt, 1H, *J=* 9.3, 2.4 Hz), 2.99 (t, 2H, J= 6.9 Hz),2.88-2.78 (m, 4H), 2.77 (t, 2H, J= 6.9 Hz); ¹³C NMR (DMSO-*d*₆) δ 164.9 (COO), 157.7 (d, *J=* 233 Hz), 132.8 (s), 127.1 (d, *J=* 10Hz), 125.1 (s), 113.1 (d, *J=* 10Hz), 112.3 (d, *J=* 5 Hz), 108.9 (d, *J=* 25 Hz), 102.9 (d, *J=* 24 Hz), 38.5 (s), 31.6 (s), 28.4(s), 25.1 (s); IR(KBr) 3055, 2916, 2850, 2472, 1581, 1454, 1211, 1180, 1045, 933, 794, 706, 597 cm⁻¹; HRMS (ESI) calculated for C₁₂H₁₅N₂FS (M⁺): 238.0943, found: 238.094, error: 1.31 ppm

### 3-(3-(2-Aminoethylthio)propyl)-5-fluoro-1H-indole oxalate (33)

The title compound has been synthesized according to the procedure for the synthesis of **32** starting from compound **16** to afford yellowish solid. ¹H NMR (DMSO-*d*₆) δ 10.97 (br s, 1H), 7.32 (dd, 1H, *J=* 8.7, 4.5 Hz), 7.26 (dd, 1H, *J=* 9.4, 2.4 Hz), 7.21 (d, 1H, *J=* 2.1 Hz), 6.88 (dt, 1H, *J=* 9.2, 2.4 Hz), 2.97 (t, 2H, *J=* 6 Hz), 2.73 (m, 4H), 2.58 (t, 2H, *J=* 7 Hz), 1.87 (m, 2H) ; ¹³C NMR (DMSO-*d*₆) δ 165.6 (COO), 157.9 (d, *J=* 233 Hz), 133.8 (s), 128.2 (d, *J=*10Hz), 125.4 (s), 114.7 (d, *J=*10Hz), 113.2 (d, *J= 5* Hz), 109.5 (d, *J=* 25 Hz), 103.6 (d, *J=* 24 Hz), 39.4 (s), 31.3 (s), 30.5 (s), 29.1 (s), 24.4 (s); IR(KBr) 3051, 2920, 2864, 2472, 1716, 1581, 1454, 1211, 1180, 1045, 933, 794, 706, 597 cm⁻¹; HRMS (ESI) calculated for C₁₃H₁₇N₂FS (M⁺): 252.1099, found: 252.1096, error: 0.84 ppm

### 5-Fluoro-3-(3-thiocyanatopropyl) 1H-indole (34)

The title compound has been synthesized according to the procedure for the synthesis of **32** starting from **16** to afford yellowish solid. ¹H NMR (CDCl₃) δ 8.15 (br s, 1H), 7.25 (dd, 1H, *J=* 8.7, 4.5 Hz), 7.22 (dd, 1H, *J=* 10.2, 2.4 Hz), 7.03 (d, 1H, *J=* 2.1 Hz), 6.95 (dt, 1H, *J=* 9.0, 2.1 Hz), 2.92 (t, 2H, *J=* 7.1 Hz), 2.87 (t, 2H, *J=* 7.3 Hz), 2.18 (m, 2H); ¹³C NMR (CDCl₃) δ 157.9 (d, *J=* 233 Hz), 133.0 (s), 127.6 (d, *J=* 9.5 Hz), 123.8 (s), 114.1 (d, *J=* 5 Hz), 112.6 (SCN), 112.1 (d, *J=* 9.5 Hz), 110.6 (d, *J=* 26 Hz), 103.7 (d, *J=* 23 Hz), 33.5 (s), 30.0 (s), 27.1 (s) ; IR (KBr) 3421, 2924, 2152, 1716, 1485, 1288, 1230, 1172, 1091, 1049, 933, 848, 794, 744, 597 cm⁻¹; HRMS (ESI) calculated for C₁₂H₁₁N₂FS (M⁺): 234.0627, found: 234.0627, error: 0.17 ppm.

### 5-Fluoro-3-(2-(N-ethylmethanesulfonamido)ethyl)-1H-indole (35)

Starting from mesylate derivative **15**, N-ethyl-3-(5-fluoroindol-3-yl)ethan-1-amine was thynsesied according to **32** and using ethylamine (40 wt% solution in water). This compound was purefied by Flash-Chromatography (BÜCHI) with cyclohexan and CH₂Cl₂ as mobile phase. The pure compound (0.5 g) was dissolved in CH₂Cl₂ (20 ml) and cooled with an ice bath. To this solution TEA (0.44 mL, 5.8 mmol) and methanesulfonyl chloride (0.77 mL, 5.5 mmol) were added. The solution was stirred at room temperature for 1/2 h. Then, it was washed with a saturated solution of NaHCO₃ and H₂O, then dried over Na₂SO₄ and filtered. The solvent was evaporated under reduced pressure. The crude material was purified by flash chromatography (BÜCHI) (Cyclohexan/ CH₂Cl₂ 4:1) to afford compound **35** as a yellow solide(yield). ¹H NMR (CDCl₃) δ 8.09 (br s, 1H), 7.25 (dd, 1H, *J=* 8.7, 4.5 Hz), 7.21 (dd, 1H, *J=* 10.2, 2.4 Hz), 7.03 (d, 1H, *J=* 2.1 Hz), 6.95 (dt, 1H, *J=* 9.0, 2.1 Hz), 3.42 (t, 2H, J= 7.5 Hz), 3.30 (q, 2H, J= 6.6 Hz), 2.81 (S, 3H, C*H*₃S), 2.75 (t, 2H, J= 7.5 Hz), 1.19 (t, 2H, *J=* 6 Hz); ¹³C NMR (CDCl₃) δ 157.0 (d, *J=* 229 Hz), 133 (ds), 127 (d, *J=* 10 Hz), 124.1 (s), 115.0 (d, *J=* 5 Hz), 112.0 (d, *J=* 10 Hz), 108.6 (d, *J=* 26 Hz), 103.6 (d, *J=* 23 Hz), 47.7 (s), 42.7 (s), 38.6 (*C*H₃S), 25.4 (s), 14.4 (s) ; IR (KBr) 3352, 2847, 1485, 1458, 1296, 1141, 775, 613 cm⁻¹; HRMS (ESI) calculated for C₁₃H₁₇N₂SO₂F (M⁺): 284.0996, found: 284.09945, error: 0.53 ppm.

### 5-Fluoro-3-(3-(N-ethylmethanesulfonamido)propyl)-1H-indole (36)

The title compound has been synthesized according to the procedure for the synthesis of 35 to afford brown solid. ¹H NMR (CDCl₃) δ 8.08 (br s, 1H, H-1), 7.25 (dd, 1H, *J=* 8.7, 4.5 Hz), 7.21 (dd, 1H, *J=* 10.2, 2.4 Hz), 7.03 (d, 1H, *J=* 2.1 Hz), 6.95 (dt, 1H, *J=* 9.0, 2.1 Hz), 3.27 (m, 4H), 2.81 (s, 3H, C*H*₃S), 2.75 (t, 2H, *J=* 7.5 Hz), 1.99 (m, 2H), 1.19 (t, 2H, *J=* 6 Hz); ¹³C NMR (CDCl₃) δ 157.0 (d, *J=* 229 Hz), 133 (s), 127 (d, *J=* 10 Hz), 124.1 (s), 115.0 (d, *J*= 5 Hz), 112.0 (d, *J=* 10 Hz), 110.5 (d, *J=* 26 Hz), 103.7 (d, *J=* 23 Hz), 47.1 (s), 42.7 (s), 38.6 (*C*H₃S), 29.0 (s), 22.3 (s), 14.4 (s) ; IR (KBr) 3352, 2847, 1485, 1458, 1296, 1141, 775, 613 cm⁻¹; HRMS (ESI) calculated for C₁₄H₁₉N₂SO₂F (M⁺): 298.1156, found: 298.1151, error: 1.68 ppm.

### 3-(2-(Ethylthio)ethyl)-5-fluoro-1H-indole (41)

The title compound has been synthesized according to the procedure for the synthesis of **32** starting from **15** to afford brown oil. ¹H NMR (CDCl₃) δ 8.07 (br s, 1H), 7.30 (dd, 1H, J= 9.9, 2.4 Hz), 7.25(dd, 1H, J= 9, 4.5 Hz), 6.96 (d, 1H, J= 2 Hz), 6.93 (dt, 1H, *J=* 9.0, 2.1 Hz), 2.86 (t, 2H, J= 7.5 Hz), 2.60 (m, 4H), 1.30 (t, 3H, J= 3 Hz); ¹³C NMR (CDCl₃) δ 157.9 (d, *J=* 229 Hz), 132.9 (s), 127.7 (d, *J=* 10 Hz), 123.7 (s), 115.3 (d, *J=* 5 Hz), 111.9 (d, *J=* 10 Hz), 110.5 (d, *J=* 26 Hz), 103.7 (d, *J=* 23 Hz), 32.3 (s), 26.3 (s), 25.9 (s), 15.0 (s) ; IR (KBr) 3421, 3309, 2924, 2850, 1481, 1454, 1172, 1026, 933, 848, 794, 748, 594 cm⁻¹; HRMS (ESI) calculated for C₁₂H₁₄NFS (M⁺): 223.0833, found: 223.08307, error: 1.03 ppm

### 3-(3-(Ethylthio)propyl)-5-fluoro-1H-indole (42)

The title compound has been synthesized according to the procedure for the synthesis of **32** starting from **16** to afford brown oil. ¹H NMR (CDCl₃) δ 8.07 (br s, 1H, H-1), 7.30 (dd, 1H, *J=* 9.9, 2.4 Hz), 7.25(dd, 1H, *J=* 9, 4.5 Hz), 7.02 (d, 1H, *J=* 2 Hz), 6.97 (dt, 1H, *J=* 9.0, 2.1 Hz), 2.79 (t, 2H, *J=* 7.5 Hz), 2.55 (q, 2H, *J=* 7.2 Hz), 2.51 (t, 2H, *J=* 7.2 Hz), 1.95 (m, 2H), 1.23 (t, 3H, *J=* 7.4 Hz); ¹³C NMR (CDCl₃) δ 157.0 (d, *J=* 229 Hz), 133.2 (s), 128.0 (d, **J=** 10 Hz), 123.9 (s), 115.6 (d, **J=** 5 Hz), 112.2 (d, **J=** 10 Hz), 110.7 (d, **J=** 26 Hz), 103.9 (d, **J=** 23 Hz), 31.7 (s), 30.2 (s), 26.4 (s), 24.5 (s), 15.3 (s) ; IR (KBr) 3421, 3309, 2924, 2850, 1481, 1454, 1172, 1026, 933, 848, 794, 748, 594 cm⁻¹; HRMS (ESI) calculated for C₁₃H₁₆NFS (M⁺): 237.0991, found: 237.0987, error: 1.69 ppm.

### 3-(2-(2-Aminoethylthio)ethyl)-7-fluoro-1H-indole oxalate (43)

The title compound has been synthesized according to the procedure for the synthesis of **32** starting from **11** to afford brown solid. ¹H NMR (DMSO-*d*₆) δ 11.32 (br s, 1H), 7.34 (d, 1H, *J* = 7.2Hz), 7.21 (d, 1H, J= 1.8 Hz), 6.94 (dt, 1H, J= 7.8, 5.1 Hz), 6.88 (dd, 1H, *J=* 11.4, 7.5 Hz), 2.99 (t, 2H, *J=* 7.2 Hz), 2.95- 2.80 (m, 4H), 2.77 (t, 2H, *J=* 7.9 Hz); ¹³C NMR (DMSO-*d*₆) δ 163.7 (COO), 149.3 (d, *J* = 241 Hz), 132.0 (d, *J* = 5 Hz), 124.8 (d, *J* = 13 Hz), 124.4 (s), 119.3 (d, *J* = 6 Hz), 115.6 (d, J= 1.9 Hz),115.3 (d, *J* = 3 Hz), 106.5 (d, *J* = 17 Hz), 39.4 (s), 31.6 (s), 28.5 (s), 25.2(s); IR (film) 3059, 2924, 2850, 1716, 1581, 1485, 1545, 1311, 1215, 1172, 1145, 768, 748, 698 cm⁻¹; HRMS (ESI) calculated for C₁₂H₁₅N₂FS (M⁺): 238.0943, found: 238.094, error: 0.7 ppm

### 3-(3-(2-Aminoethylthio)propyl)-7-fluoro-1H-indole oxalate (44)

The title compound has been synthesized according to the procedure for the synthesis of **32** starting from **12** to afford brown solid. ¹H NMR (DMSO-*d*₆) δ 11.32 (br s, 1H), 7.34 (d, 1H, *J* = 7.2Hz), 7.21 (d, 1H, J=1.8 Hz), 6.98-6.84 (m, 2H), 2.97 (t, 2H, *J=* 6.9 Hz), 2.81-2.70 (m, 2H), 2.58 (t, 2H, *J=* 7.2 Hz), 1.89 (m, 2H) ; ¹³C NMR (DMSO-*d*₆) δ 163.7 (COO), 149.3 (d, *J* = 241 Hz), 131.2 (d, *J* =5.9 Hz), 124.0 (d, *J =* 13 Hz), 123.6 (s), 118.5 (d, *J* = 6.1 Hz), 114.8 (d, J= 1.9 Hz), 114.5 (d, *J =* 3.1 Hz), 105.7 (d, *J* = 16.1 Hz), 38.6 (s), 30.5 (s), 29.7 (s), 28.1 (s), 23.6 (s) ; IR (film) 3059, 2924, 2850, 1716, 1581, 1485, 1545, 1311, 1215, 1172, 1145, 768, 748, 698 cm⁻¹; HRMS (ESI) calculated for C₁₃H₁₇N₂FS (M⁺): 252.1099, found: 252.1096, error: 1.29 ppm

### 3-(3-N-Hydroxylaminopropyl)-5-fluoro-1H-indole (45)

The title compound has been synthesized according to the procedure for the synthesis of **32** starting from **16** and using hydroxylamine instead of cysteamine to afford yellow solid. ¹H NMR (DMSO-*d*₆) δ 10.9 (br s, 1H), 7.33 (m, 3H), 6.98 (dt, 1 H, *J=* 7.4, 0.8 Hz), 3.10 (t, 2H, *J=* 9 Hz), 2.71 (m, 2H), 1.93 (m, 2H); ¹³C NMR (DMSO-*d*₆) δ 164.9 (C-oxalate), 157.9 (d, *J*= 232 Hz), 133.8 (s), 128.1 (d, *J*= 10 Hz), 125.5 (s), 114.0 (d, *J*= 5 Hz), 113.1 (d, *J*= 10 Hz), 109.2 (d, *J*= 25 Hz), 103.8 (d, *J*= 23 Hz), 51.1 (s), 25.0 (s), 22.4 (s); IR (KBr) 3421, 3055, 2924, 2854, 1716, 1627, 1485, 1454, 1168, 1091, 933, 790, 698, 597 cm⁻¹; HRMS (ESI) calculated for C₁₁H₁₃N₂F (M⁺): 208.1018, found: 208.1012, error: 2.88 ppm.

### 5-Fluoro-3-propyl-1H-indole (46)

To a solution of mesylate derivative **16** (1 g, 3.6 mmol) in dioxane (5 mL) finely powdered KOH in methanol (15 ml) was added. The reaction medium was refluxing for 4 h. After cooling, the mixture was extracted with EtOAc (30 mL). The organic layer was washed three times by H₂O, dried over Na₂SO₄ and evaporated to dryness to afford a crude product. The compound was purified by colomn chromatography using CH₂Cl₂ as mobile phase to afford yellow oil. ¹H NMR (CDCl₃) δ 7.88 (br s, 1H), 7.24 (dd, 1H, J= 8.7, 2.1 Hz), 7.21 (dd, 1H, J= 8.7, 4.8 Hz), 6.97 (d, 1H, *J=* 2.1 Hz), 6.95 (dt, 1H, *J=* 9.0, 2.7 Hz), 2.66 (t, 2H, *J=* 7.5 Hz), 1.71 (m, 2H), 0.98 (t, 3H, J= 7.4 Hz); ¹³C NMR (CDCl₃) δ 158.0 (d, *J=* 229 Hz), 133.2 (s), 128.4 (d, *J=* 10 Hz), 123.4 (s), 117.4 (d, *J=* 5 Hz), 111.9 (d, *J=* 10 Hz), 110.4 (d, *J=* 26 Hz), 104.3 (d, *J=* 23 Hz), 27.4 (s), 23.4 (s), 14.3 (s) ; IR (KBr) 3421, 2924, 2152, 1716, 1485, 1288, 1230, 1172, 1091, 1049, 933, 848, 794, 744, 597 cm⁻¹; HRMS (ESI) calculated for C₁₁H₁₂NF (M⁺): 177.0961, found: 177.09535, error: 4.24 ppm.

### 3-(4-Butanenitrile)-5-fluoro-1H-indole (50)

To a solution of NaCN (2 g, 41 mmol) in H₂O (10 mL), DMAc (10 mL) was added and heated at 100 °C. Then, a solution of **16** (2 g, 7.4 mmol) in DMAc (10 mL) was added dropwise. The reaction was stirred at this temperature for 2 h, then cooled to room temperature and extracted with EtOAc. The organic layer was washed with water, dried on Na₂SO₄ and evaporated. The residue was purified by flash chromatography and yielded brown oil (1 g, 75% yield). IR (film) 3380, 3125, 2930, 2240, 1716, 1634, 1575, 1496, 1448, 1357, 1237, 1090, 1044, 964, 782, 726 cm-¹.

### 3-(3-(propylamido))- 7-fluoro-1H-indole 53

A nitrile derivative **47**, 3-(3-Propanenitrile)-7-fluoro-*1H*-indole, was synthesised starting from **11** according to synthesis procedure of **50.** 3-(3-Propanenitrile)-7-fluoro-*1H*-indole was dissolved in (40 mL) of *t*-BuOH, to this solution finely powdered KOH (5 g, 89 mmol) was added. The solvent was refluxed for 2 h with stirring. Then a saturated solution of NaCl (50 mL) was added and the resulting mixture was extracted 3 times with CH₂Cl₂. Flash chromatography was performed (CH₂Cl₂/EtOAc 4:1 then CH₂Cl₂/methanol 1:1). The fractions obtained with the second solvent mixture were evaporated and title compound was obtained brown solid. ¹H NMR (CD₃OD) δ 7.33 (d, 1H, *J=* 8.1 Hz), 7.08 (s), 6.92 (dt, 1H, J= 7.8, 4.8 Hz), 6.80 (dd, 1H, *J=* 11.1, 7.8 Hz), 3.04 (t, 2 H *J*= 7.2 Hz), 2.57 (t, 2 H, *J*=8.1 Hz); ¹³C NMR (CD₃OD) δ 178.9 (CO), 151.2 (d, *J*= 241 Hz), 132.6 (d, *J* = 5.7 Hz), 126.1 (d, *J =* 13 Hz), 124.1 (s), 119.8 (d, *J* = 6 Hz), 116.7 (d, *J* = 2 Hz), 115.4 (d, *J* = 3 Hz), 106.9 (d, *J =* 16 Hz), 37.6 (s), 22.5 (s); IR (KBr) 3423, 2880, 1640, 1581, 1485, 1230, 1171, 775, 613 cm⁻¹;

### 3-(4-(butylamido))- 7-fluoro-1H-indole 54

The title compound has been synthesized according to the procedure for the synthesis of **53** to afford brown solid. ¹H NMR (CD₃OD) δ 7.31 (d, 1H, *J=* 8.1 Hz), 7.06 (s), 6.92 (dt, 1 H, J= 7.8, 4.8 Hz), 6.80 (dd, 1 H, *J=* 11.1, 7.8 Hz), 2.76 (t, 2 H *J=* 7.5 Hz), 2.26 (t, 2 H, *J=* 7.8 Hz), 1.99 (m, 2 H); ¹³C NMR (CD₃OD) δ 179.3 (CO), 151.3 (d, *J=* 241 Hz), 132.9 (d, *J* = 5.7 Hz), 126.1 (d, *J* = 13 Hz), 124.1 (s), 119.8 (d, *J* = 6 Hz), 116.7 (d, *J* = 2 Hz), 115.5 (d, *J* = 3 Hz), 106.9 (d, *J =* 16 Hz), 36.3 (s), 27.7 (s), 25.8 (s); IR (KBr) 3423, 2880, 1640, 1581, 1485, 1230, 1171, 775, 613 cm⁻¹;

### 3-(3-(propylamido))- 5-fluoro-1H-indole (55)

The title compound has been synthesized according to the procedure for the synthesis of **53** to afford brown solid (1.7 g, 70% yield). ¹H NMR (CD₃OD) δ 7.27 (dd, 1H, *J*= 9.0, 4.5 Hz), 7.17 (dd, 1 H, *J=* 9.9, 2.1 Hz), 7.07 (s, 1 H), 6.83 (dt, 1 H, *J=* 9.3, 2.4 Hz), 3.04 (t, 2 H *J=* 7.2 Hz), 2.57 (t, 2 H, *J=* 8.1 Hz); ¹³C NMR (CD₃OD) δ 179.5 (CO), 158.8 (d, *J*= 230 Hz), 134.9 (s), 129.1 (d, *J*= 10 Hz), 124.9 (s), 116.6 (d, *J*= 5 Hz), 112.8 (d, *J=* 10 Hz), 110.3 (d, *J=* 25 Hz), 103.9 (d, *J=* 23 Hz), 37.6 (s), 22.5 (s); IR (KBr) 3423, 2880, 1640, 1581, 1485, 1230, 1171, 775, 613 cm⁻¹;

### 3-(4-(Butylamido))-5-fluoro-1H-indole 56

The title compound has been synthesized according to the procedure for the synthesis of **53** to afford white crystals. ¹H NMR (DMSO-*d*₆) δ 10.88 (br s, 1H), 7.30 (dd, 1H, *J=* 8.7, 4.5 Hz), 7.27 (dd, 1 H, *J=* 9.1, 2.4 Hz), 7.21 (d, *J=* 1.2 Hz, 1 H), 6.91 (dt, 1 H, *J=* 7.4, 0.8 Hz), 2.65 (t, 2 H *J*= 7.6 Hz), 2.13 (t, 2 H, *J*= 7.8 Hz), 1.85 (m, 2 H); ¹³C NMR (DMSO-*d*₆) δ 174.9 (CO), 157.9 (d, *J*= 232 Hz), 133.1 (s), 127.8 (d, *J*= 10 Hz), 124.9 (s), 115.1 (d, *J*= 5 Hz), 112.7 (d, *J*= 10 Hz), 110.5 (d, *J*= 25 Hz), 103.7 (d, *J*= 23 Hz), 35.4 (s), 26.4 (s), 24.8 (s); IR (film) 3423, 2880, 1640, 1581, 1485, 1230, 1171, 1055 cm⁻¹; HRMS (ESI) calculated for C₁₂H₁₃N₂FO (M⁺): 220.1012, found: 220.10117, error: 0.14 ppm.

### 3-(5-(pentylamido)) -7-fluoro-1H-indole 57

The title compound has been synthesized according to the procedure for the synthesis of **35** to afford brown solid. ¹H NMR (CD₃OD) δ 7.31 (d, 1H, *J*= 8.1 Hz), 7.05 (s), 6.92 (dt, 1 H, J= 7.8, 4.8 Hz), 6.80 (dd, 1 H, *J*= 11.1, 7.8 Hz), 2.77 (t, 2 H *J*= 6.6 Hz), 2.24 (t, 2 H, *J*= 7.2 Hz), 1.72 (m, 4 H); ¹³C NMR (CD₃OD) δ 179.4 (CO), 151.3 (d, *J*= 241 Hz), 132.9 (d, *J* = 5.7 Hz), 126.1 (d, *J* = 13 Hz), 123.9 (s), 119.6 (d, *J* = 6 Hz), 116.7 (d, *J* = 2 Hz), 115.5 (d, *J* = 3 Hz), 106.8 (d, *J* = 16 Hz), 36.5 (s), 31.1 (s), 26.9 (s), 26.0 (s); IR (KBr) 3423, 2880, 1640, 1581, 1485, 1230, 1171, 775, 613 cm⁻¹;

### 3-(5-(pentylamido)) -5-fluoro-1H-indole 58

The title compound has been synthesized according to the procedure for the synthesis of **53** to afford brown solid. ¹H NMR (CD₃OD) δ 7.27 (dd, 1H, *J=* 9.0, 4.5 Hz), 7.17 (dd, 1 H, *J=* 9.9, 2.1 Hz), 7.07 (s, 1 H), 6.83 (dt, 1 H, *J=* 9.3, 2.4 Hz), 2.73 (t, 2 H *J=* 6.6 Hz), 2.25 (t, 2 H, *J*= 6.9 Hz), 1.71 (m, 4H); ¹³C NMR (CD₃OD) δ 179.5 (CO), 158.8 (d, *J*= 230 Hz), 134.9 (s), 129.1 (d, *J*= 10 Hz), 124.9 (s), 116.6 (d, *J*= 5 Hz), 112.8 (d, *J=* 10 Hz), 110.3 (d, *J*= 25 Hz), 103.9 (d, *J*= 23 Hz), 36.6 (s), 31.1 (s), 26.9 (s), 25.9 (s); IR (film) 3423, 2880, 1640 , 1581, 1485, 1230, 1171, 1055 cm⁻¹.

### 3-(4-(N-methyl, butylamido))-5-fluoro-1H-indole 59

To a solution of **50** (2 g, 9.9 mmol) in *t*-BuOH (40 mL) was added finely powdered KOH (5 g, 89 mmol). The solvent was refluxed for 2 h with stirring. Then methyl bromide (20 mL) was added to the reaction mixture and stirred for 1 hour. The compound was extracted three times with ethyl acetate, dried with Na₂SO₄ and evaporated. Flash chromatography was performed CH₂Cl₂:EtOAc (4:1) then CH₂Cl₂:methanol (1:1). The fractions obtained with the second solvent mixture were evaporated and title compound was obtained as green crystals. ¹H NMR (CD₃OD) δ 7.27 (dd, 1H, *J=* 9.0, 4.8 Hz), 7.20 (dd, 1 H, *J=* 9.6, 2.7 Hz), 7.09 (s, 1 H), 6.89 (dt, 1 H, *J=* 9.3, 2.4 Hz), 3.02 (s, 3H, CH₃), 2.74 (t, 2 H *J=* 7.5 Hz), 2.27 (t, 2 H, *J=* 7.5 Hz), 1.98 (m, 2H); ¹³C NMR (CD₃OD) δ 179.3 (CO), 158.8 (d, *J*= 230 Hz), 134.4 (s), 129.8 (d, *J*= 10 Hz), 124.9 (s), 115.6 (d, *J*= 5 Hz), 111.1 (d, *J*= 10 Hz), 110.3 (d, *J*= 25 Hz), 104.5 (d, *J=* 23 Hz), 40.32 (CH₃), 37.5 (s), 27.8 (s), 25.7 (s); IR (KBr) 3423, 2880, 1640, 1581, 1485, 1230, 1171, 775, 613 cm⁻¹;

### 3-(4-(N-ethyl, butylamido))-5-fluoro-1H-indole 60

The title compound has been synthesized according to the procedure for the synthesis of **59** by using ethyl bromide instead of methyl bromide to afford brown solid. ¹H NMR (CD₃OD) δ 7.29 (dd, 1H, *J=* 9.0, 4.8 Hz), 7.19 (dd, 1 H, *J=* 9.6, 2.7 Hz), 7.09 (s, 1 H), 6.89 (dt, 1 H, *J=* 9.3, 2.4 Hz), 4.14 (q, 2H, CH₃C*H*₂), 2.74 (t, 2 H *J*= 7.5 Hz), 2.27 (t, 2 H, *J=* 7.5 Hz), 1.98 (m, 2H), 1.40 (t, 3H, *J=* 7.2 Hz, C*H*₃CH₂); ¹³C NMR (CD₃OD) δ 179.3 (CO), 158.8 (d, *J*= 230 Hz), 134.3 (s), 129.9 (d, *J=* 10 Hz), 124.9 (s), 115.6 (d, *J*= 5 Hz), 111.1 (d, *J*= 10 Hz), 110.4 (d, *J*= 25 Hz), 104.5 (d, *J=* 23 Hz), 41.9 (CH₃*C*H₂), 36.3 (s), 27.8 (s), 25.7 (s), 16.0 (*C*H₃CH₂); IR (KBr) 3423, 2880, 1640, 1581, 1485, 1230, 1171, 775, 613 cm⁻¹;

**Myeloperoxidase inhibition (MPO) assay procedure**

The assay was based on the production of taurine chloramine produced by the MPO/H₂O₂/Cl- system in the presence of a selected inhibitor at defined concentration. The reaction mixture contained the following reagents in a final volume of 200 µL: pH 7.4 phosphate buffer (10 mM PO₄³⁻/300 mM NaCl), taurine (15 mM), the compound to be tested (up to 20 µM), and the fixed amount of the recombinant MPO (6.6 µL of MPO batch solution diluted 2.5 times, 40 nM). When necessary, the volume was adjusted with water. This mixture was incubated at 37 °C and the reaction initiated with 10.0 µL of H₂O₂ (100 µM). After 5 min, the reaction was stopped by the addition of 10 µL of catalase (8 U/µL). To determine the amount of taurine chloramines produced, 50 µL of 1.35 mM solution of thionitrobenzoic acid were added and the volume adjusted to 300 µL with water. Then, the absorbance of the solutions was measured at 412 nm with a microplate reader and the curve of the absorbance as a function of the inhibitor concentration was plotted. IC₅₀ values were then determined using standard procedures taking into account the absence of hydrogen peroxide as 100% of inhibition and the absence of inhibitors as 0% of inhibition.

### Low-density lipoproteins oxidation inhibition

Preparation of the Recombinant Enzyme and of LDL

Recombinant MPO was prepared as previously described. Each batch solution is characterized by its protein concentration (mg/mL), its activity (U/mL), and its specific activity (U/mg). The chlorination activity was determined according to Hewson and Hager. Human plasma served for the isolation of LDL by ultracentrifugation according to Havel et al. Before oxidation, the LDL fraction (1.019 < d < 1.067 g/mL) was desalted by two consecutive passages through PD10 gel-filtration columns (Amersham Biosciences, The Netherlands) using PBS buffer. The different steps were carried out in the dark, and the protein concentration was measured by the Lowry assay for both MPO and LDL.

Inhibition of LDL Oxidation

LDL oxidation was carried out at 37 °C in a final volume of 500 µL. The reaction mixture contained the following reagents at the final concentrations indicated between brackets: pH 7.2, PBS buffer, MPO (1 µg/mL), LDL (1000 µg/mL), 2 µL 1 N HCl (4 mM), one of the drugs at different concentrations, and H₂O₂ (100 µM). The reaction was stopped after 5 min by cooling the tubes in ice. The assay was performed as described by Moguilevsky et al. in a NUNC maxisorp plate (VWR, Zaventem, Belgium): 200 ng/well of LDL was coated overnight at 4 °C in a sodium bicarbonate pH 9.8 buffer (100 µL) [47] Afterward, the plate was washed with TBS 80 buffer and then saturated during 1 h at 37 °C with the PBS buffer containing 1% BSA (150 µL/well). After washing the wells twice with the TBS 80 buffer, the monoclonal antibody Mab AG9 (200 ng/well) obtained according to a standard protocol and as previously described was added as a diluted solution in PBS buffer with 0.5% BSA and 0.1% of Polysorbate 20. After incubation for 1 h at 37 °C, the plate was washed four times with the TBS 80 buffer and a 3000 times diluted solution of IgG antimouse alkaline phosphatase (Promega, Leiden, The Netherlands) in the same buffer was added (100 µL/well). The wells were washed again four times and a revelation solution (150 µL/well) containing 5 mg of para-nitrophenyl phosphate in 5 mL of diethanolamine buffer was added for 30 min at room temperature. The reaction was stopped with 60 µL/well of 3 N NaOH solution. The measurement of the absorbance was performed at 405 nm with a background correction at 655 nm with a Bio-Rad photometer for a 96-well plate (Bio-Rad laboratories, CA, USA). Results were expressed as IC₅₀.

### Assesment of 5-HT reuptake inhibition.

Inhibition assessment of 5-HT reuptake was performed as follows. The mammalian expression plasmid pcDNA3.1 containing human 5-HT transporter (hSERT) cDNA has been described previously. HEK-293 MSR cells (Invitrogen) were cultured as monolayers in Dulbecco's modified Eagle's medium (BioWhitaker) supplemented with 10% fetal calf serum (Invitrogen), 100 units/mL penicillin, 100 µg/mL streptomycin (BioWhitaker) and 6 µg/mL Geneticin (Invitrogen) at 95% humidity, 5% p(CO₂) and 37°C. Cells were detached from the culture flasks by Versene (Invitrogen) and trypsin/EDTA (BioWhitaker) treatment for subculturing or seeding into white TC-microtiter plates (Nunc). Transfection and measurement of [³H]5-HT (PerkinElmer Life Sciences) uptake was performed as described by Larsen et al. (2004) except that HEK-293 MSR cells (Invitrogen) were used instead of COS-1 cells.

### Example 1

The inhibition of myeloperoxidase enzyme was evaluated in presence of various compounds. The lower is the IC₅₀ value, the better is the inhibition of myeloperoxidase enzyme. Results are reported in table 1.

**Table 1**

| Comp. | Form. | n | R¹ | R² | R³ | R⁶ | R⁷ | R⁸ | R⁹ | R¹⁰ | R¹¹ | IC₅₀ (nM) | Serotonin Ki (nM) |
|---|---|---|---|---|---|---|---|---|---|---|---|---|---|
| Prior art | (IIIa) | 2 | H | H | -NH₂ | F | H | H | H | - | - | 200 | 10-20 |
| Prior art | (IIIa) | 3 | H | H | -NH₂ | F | H | H | H | - | - | 50 | 10-20 |
| Prior art | (IIIa) | 4 | H | H | -NH₂ | F | H | H | H | - | - | 15 | 10-20 |
| 30 | (IIIa) | 4 | H | H | -NH₂ | H | F | H | H | - | - | 6 | - |
| 32 | (IV) | 2 | H | H | -S-(CH₂)₂-NH₂ | F | H | - | - | - | - | 4 | - |
| 43 | (IV) | 2 | H | H | -S-(CH₂)₂-NH₂ | H | F | - | - | - | - | 3 | 25 |
| 34 | (IV) | 3 | H | H | -SCN | F | H | - | - | - | - | 5 | 212 |
| 35 | (IV) | 2 | H | H | 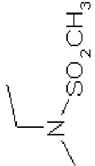 | F | H | - | - | - | - | 14 | 174 |
| 45 | (IV) | 3 | H | H | -NHOH | F | H | - | - | - | - | 11 | 20 |
| 56 | (V) | 3 | H | H | - | F | H | - | - | H | H | 18 | 631 |

Compound 30 exhibits excellent inhibition of myeloperoxidase enzyme. Compared to the prior art, compound 30 differs in the position of fluor atom on indole ring, i.e. fluor being at position 7 instead of 5 on indole ring. This modification of the backbone leads to enhanced properties with respect to the inhibition of myeloperoxidase enzyme. Indeed, the prior art (WO 2010/136546, noted "Prior art" in table 1 above) mentions that compounds, with F atom at position 5 on the indole ring and wherein n is 2, 3 or 4, has an IC₅₀ of 200, 50 and 15 nM respectively. The compounds of the present invention allow decreasing the value of IC₅₀ by changing the position of the fluor atom on the indole ring. This behavior is confirmed with other compounds having similar formula. For example, compounds 26 and 27 having the same formula than compound 30, except that n is 2 and 3 respectively, show enhanced inhibition properties against myeloperoxidase enzyme than their corresponding compound wherein the fluor atom is at position 5 on the indole ring.

Results disclosed in table 1, further highlight that by changing the functional group of the lateral alkyl chain, the inhibition of myeloperoxidase enzyme can be improved. Indeed, introducing functional groups such as thiocyanate, substituted thioalkyl, hydroxylamine or amino sulfone derivatives, IC₅₀ values lower than 15 nM can be achieved, which is a breakthrough compared to the prior art. In particular, IC₅₀ values lower than 5 nM can be obtained for compounds 32 and 34 bearing -S-(CH₂)₂-NH₂ or thiocyanate moiety respectively.

The inhibition of myeloperoxidase enzyme is also further enhanced when the side chain R³ is an amido. For example, compound 56 has IC₅₀ value of 18 nM while the corresponding compound with an amino group has a value of 50nM (WO 2010/136546, table 2).

The serotonin reuptake inhibition was evaluated in presence of various compounds according to the present invention. Results are reported in table 1 above. The serotonin reuptake inhibition was also evaluated for compounds of the prior art. Compounds of the prior art exhibit Ki values between 10 to 20 nM which means that these compounds have better affinity to serotonin receptor than myeloperoxidase enzyme. This could lead to severe secondary indications to patient. In contrast compounds of the present invention shows better affinity for MPO inhibition than affinity for serotonin receptor. In particular, compounds 56 bearing an amido functional group exhibit excellent diffentiated affinity between MPO and serotonin receptor (Ki = 631 nM). This behavior was totally unexpected in view of the prior art. The same behavior was also observed for other compounds bearing functional group such as thiocyanate (compound 34, Ki = 212 nM), amino sulfone derivatives (compound 35, Ki = 174nM), or thioalkyl derivatives (compound 43, Ki = 25 nM). Compounds bearing hydroxylamine functional group show also better affinity for MPO than serotonin receptor (compound 45, Ki = 20 nM compared to IC₅₀ of 11 nM) Hence, compounds according to the present invention shows improved and/or novel properties compared to compounds known in the prior art, in order to improve inhibition of myeloperoxidase enzyme and/or lowering serotonin receptor affinty. Pharmaceutical compositions comprising compounds according to the present invention have excellent therapeutic effect for use as medicine, and in particular for treating neurologic and/or inflammatory diseases or disorders.

### Example 2

The inhibition of LDL oxidation was evaluated in presence of various compounds. Results are reported in table 2.

**Table 2**

| Compound | Formula | n | R¹ | R² | R³ | R⁶ | R⁷ | R⁸ | R⁹ | R¹⁰ | R¹¹ | IC₅₀ (nM) |
|---|---|---|---|---|---|---|---|---|---|---|---|---|
| 30 | (IIIa) | 4 | H | H | -NH₂ | H | F | H | H | - | - | 21 |
| 32 | (IV) | 2 | H | H | -S-(CH₂)₂-NH₂ | F | H | - | - | - | - | 8 |
| 33 | (IV) | 3 | H | H | -S-(CH₂)₂-NH₂ | F | H | - | - | - | - | 21 |
| 34 | (IV) | 3 | H | H | -SCN | F | H | - | - | - | - | 23 |
| 35 | (IV) | 2 | H | H | 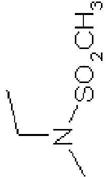 | F | H | - | - | - | - | 27 |
| 36 | (IV) | 3 | H | H | 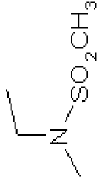 | F | H | - | - | - | - | 27 |
| 43 | (IV) | 2 | H | H | -S-(CH₂)₂-NH₂ | H | F | - | - | - | - | 6 |
| 45 | (IV) | 3 | H | H | -NHOH | F | H | - | - | - | - | 21 |
| 54 | (V) | 3 | H | H | - | H | F | - | - | H | H | 24 |
| 56 | (V) | 3 | H | H | - | F | H | - | - | H | H | 18 |

Compounds of the present invention show also excellent activity towards inhibition of LDL oxidation. IC₅₀ values lower than 30nM and even lower than 10 nM were observed (see table 2, compounds 32 et 43). Inhibition of LDL oxidation can therefore be further improved compared to the prior art. Introducing new functional groups such as thiocyanate, substituted thioalkyl, hydroxylamine, amido or amino sulfone derivatives, or changing the position of the fluor atom on the indole ring can positively influence the LDL oxidation inhibition. It is further noted that compound 56 bearing amido functional group has both excellent properties for inhibiting myeloperoxidase enzyme and LDL oxidation. Indeed, in both cases IC₅₀ values observed was 18 nM and, in addition compound 56 shows low affinty for serotonin receptor (Ki = 631 nM).

### Example 3

Compound 56 inhibits myeloperoxidase enzyme and oxidation of LDL as showed above in table 1 and table 2. Various 3-amidoindole analogues were prepared and most of them have the same properties with respect to the inhibition of myeloperoxidase enzyme (MPO IC₅₀) and/or of oxidation of LDL (Mox LDL). Results are listed in table 3 below.

**Table 3**

| Compound | Formula | n | R¹ | R² | R⁶ | R⁷ | R¹⁰ | R¹¹ | MPO IC₅₀ (nM) | Mox LDL IC₅₀ (nM) |
|---|---|---|---|---|---|---|---|---|---|---|
| 54 | (V) | 3 | H | H | H | F | H | H | 26 | 24 |
| 56 | (V) | 3 | H | H | F | H | H | H | 18 | 18 |
| 57 | (V) | 4 | H | H | F | H | H | H | 39 | 43 |
| 58 | (V) | 4 | H | H | H | F | H | H | 35 | 44 |
| 59 | (V) | 3 | H | H | F | H | CH₃ | H | 40 | 95 |

Compound 56 has low affinity with serotonin receptor (table 1, Ki = 631nM). The compounds prepared in this example show similar performance with regard to serotonin receptor. Hence, 3-amidoindole analogues of formula (V) according to the present invention are excellent candidates for use as medicine and in particular for use in the treatment of neurologic and/or inflammatory diseases or disorders.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. Compounds, or a pharmaceutically acceptable salt, solvate, hydrate, polymorph, co-crystal thereof, wherein said compound is of formula A-B (I) wherein
A is substitued or not aromatic N-heterocycle; and
B is -[C(R¹)(R²)]ₙ-R³ wherein
n is an integer between 1 and 10,
R¹ and R² represent independently in each of the n units a substituent selected from the group consisting of hydrogen, substituted or not C₁-C₁₀ alkyl, halogen, oxy derivatives, thioderivatives, substituted or not C₃-C₁₀ cycloalkyl, substituted or not C₆-C₁₈ aryl, substituted or not C₂-C₁₀ alkenyl, substituted or not C₂-C₁₀ alkynyl, R³ represents a substituent selected from the group consisting of hydrogen, substituted or not C₁-C₁₀ alkyl, halogen, substituted or not C₃-C₁₀ cycloalkyl, substituted or not C₆-C₁₈ aryl, substituted or not C₂-C₁₀ alkenyl, substituted or not C₂-C₁₀ alkynyl, amino, heterocycle, thiocyanate, thio derivative, hydroxylamine, amido, sulfonamide, amino sulfone derivatives, ether, thioether, ester, thioester, azido, azo, cyano, nitro, nitrooxy, carbamate, thiocarbamate, sulfinyl derivative, sulfonyl derivatives, acyl derivative, oxy derivative, ureido, thioureido, imino, oximino, hydrazino, thioamido, carboxylic, phosphate, phosphonate, carbamoyl phosphate;
with the proviso that when A is 5-fluoroindole, B is bonded at position 3 of the 5-fluoroindole moiety, R¹ is hydrogen and n is between 2 and 10, R³ is not -NR⁴R⁵ wherein R⁴ and R⁵ independently represent a substituent selected from the group consisting of hydrogen, C₁-C₁₀ alkyl, C₃-C₁₀ cycloalkyl, aminoalkyl, or R⁴ and R⁵ taken together with the nitrogen atom to which they are attached to form a four to ten-membered heterocycle, and R² is not independently in each of the n units a substituent selected from the group consisting of hydrogen, C₁-C₁₀ alkyl, halogen, alkoxy, aminoalkyl, and alkylamino;
for the treatment or the prophylaxis of neurologic and/or inflammatory diseases or disorders.

2. Compounds according to claim 1 wherein aromatic N-heterocycle is selected from the group consisting of indole, imidazole, quinoline, isoquinoline, pyridine, triazine, pyrazine, pyrimidine, pyridazine, isoindole, pyrrole, benzimidazole, purine, pyrazole, indazole, quinoxaline, acridine, quinazoline, benzopyrazole, indolizine, carbazole and cinnoline.

3. Compounds according to claim 1 or 2 wherein A is of general formula (IIa), (IIb), (IIc), (IId), (IId), (IIe), (IIf), or (IIg) 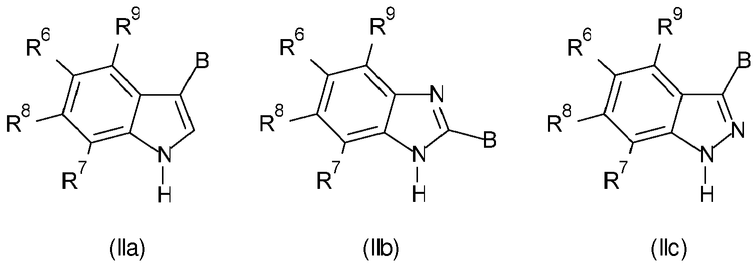 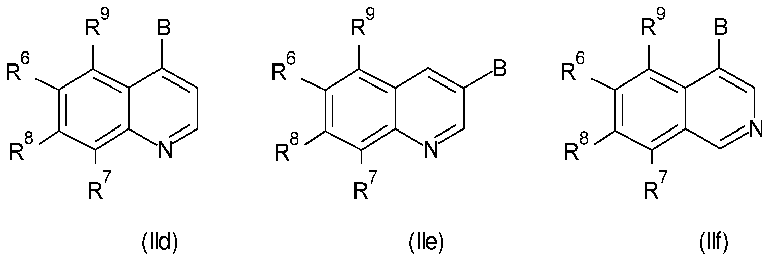 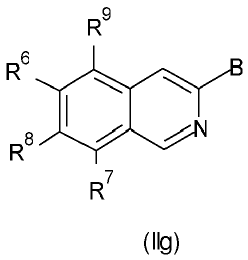 wherein R⁶, R⁷, R⁸ and R⁹ independently represent a substituent selected from the group consisting of hydrogen, F, Cl, Br, CO₂H, CN and C₁-C₁₀ alkoxy.

4. Compounds according to any of the previous claims wherein said compounds have general formula (IIIa) 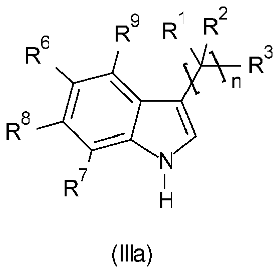 wherein n is an integer between 1 and 10,
R⁶, R⁷, R⁸ and R⁹ independently represent hydrogen, fluoride, chloride, bromide, - CO₂H, CN, C₁-C₁₀ alkoxy;
R¹ and R² represent independently in each of the n units a substituent selected from the group consisting of hydrogen, substituted or not C₁-C₁₀ alkyl;
R³ represents a substituent selected from the group consisting of substituted or not C₁-C₁₀ alkyl, substituted or not C₃-C₁₀ cycloalkyl, substituted or not C₆-C₁₈ aryl, amino, heterocycle, substituted or not thioalkyl, thiocyanate, cyano, hydroxylamine, amido, sulfonamide, amino sulfone derivatives, ether, thioether, thioester, ester, carboxylic, sulfinyl derivative, acyl derivative, oxy derivative.

5. Compounds according to any of the previous claim wherein R³ represents a substituent selected from the group consisting of substituted or not C₁-C₁₀ alkyl, amino, substituted or not thioalkyl, thiocyanate, amino sulfone derivatives, substituted or not piperidine, hydroxylamine, amido, cyano.

6. Compounds according to any of the previous claim having general formula (IV) 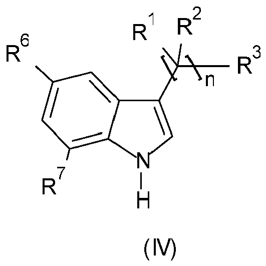 wherein n is an integer between 1 and 10,
R⁶ and R⁷ independently represent hydrogen, F, Br, Cl, CO₂H, CN, C₁-C₁₀ alkoxy;
R¹ and R² represent independently in each of the n units a substituent selected from the group consisting of hydrogen, substituted or not C₁-C₁₀ alkyl;
R³ represents a substituent selected from the group consisting of thiocyanate, cyano, amino sulfone derivatives, amido, substituted or not thioalkyl, hydroxylamine;
or a pharmaceutically acceptable salt, solvate, hydrate, polymorph, co-crystal thereof, for the treatment or the prophylaxis of neurologic and/or inflammatory diseases or disorders.

7. Compounds according to any of the previous claim having general formula (V) 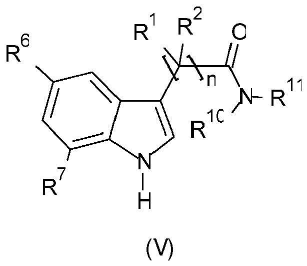 wherein n is an integer between 1 and 10;
R⁶ and R⁷ independently represent hydrogen, F, Br, Cl, CO₂H, CN, C₁-C₁₀ alkoxy;
R¹ and R² represent independently in each of the n units a substituent selected from the group consisting of hydrogen and substituted or not C₁-C₁₀ alkyl, preferably hydrogen and substituted or not C₁-C₆ alkyl, more preferably hydrogen and substituted or not C₁-C₂ alkyl;
R¹⁰ and R¹¹ independently represent a substituent selected from the group consisting of hydrogen, substituted or not C₁-C₁₀ alkyl, C₂-C₁₀ alkenyl, C₂-C₁₀ alkynyl, C₆-C₁₈ aryl, heterocycle, or R¹⁰ and R¹¹ taken together with the nitrogen atom to which they are attached form a four to ten membered heterocycle;
or a pharmaceutically acceptable salt, solvate, hydrate, polymorph, co-crystal thereof, for the treatment or the prophylaxis of neurologic and/or inflammatory diseases or disorders.

8. Compounds according to any of claims 1 to 5 having general formula (IV) 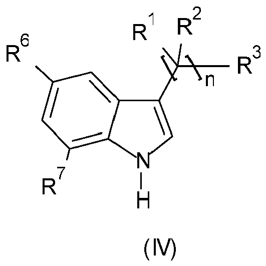 wherein n is an integer between 1 and 10;
R⁶ represents hydrogen; R⁷ represents F;
R¹ and R² represent independently in each of the n units a substituent selected from the group consisting of hydrogen, substituted or not C₁-C₁₀ alkyl; preferably hydrogen and substituted or not C₁-C₆ alkyl, more preferably hydrogen and substituted or not C₁-C₂ alkyl; R³ is amino;
for the treatment or the prophylaxis of neurologic and/or inflammatory diseases or disorders.

9. Pharmaceutical composition comprising a therapeutically effective amount of a compound according to any one of the claims 1 to 8, or a pharmaceutically acceptable salt, solvate, hydrate, polymorph, co-crystal thereof, in admixture with a pharmaceutically acceptable adjuvant, diluent or carrier.

10. Pharmaceutical composition according to claim 9, for the treatment or prophylaxis of neurologic and/or inflammatory diseases or disorders.

11. An in vitro method for inhibiting myeloperoxidase enzyme activity **characterised in that** said method comprises the step of adding a compound according to any one of claims 1 to 8 to a medium containing said enzyme, said compound being added in a concentration effective to inhibit the activity of said enzyme.

12. Compound according to any one of claims 1 to 8 or pharmaceutical composition according to claims 9 or 10 for inhibiting myeloperoxidase enzyme activity, for inhibiting low density lipoproteins oxidation.

13. An in vitro method for inhibiting low density lipoproteins (LDL) oxidation **characterised in that** said method comprises the step of contacting a compound according to any one of claims 1 to 8 in a medium containing said low density lipoproteins and myeloperoxidase enzyme.

14. Compounds having general formula (IV) 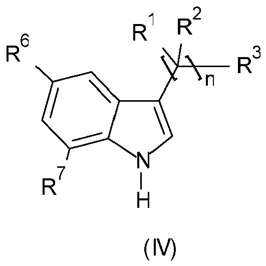 wherein n is an integer between 1 and 10,
R⁶ and R⁷ independently represent hydrogen, F, Br, Cl, CO₂H, CN, C₁-C₁₀ alkoxy; R¹ and R² represent independently in each of the n units a substituent selected from the group consisting of hydrogen and C₁-C₁₀ alkyl;
R³ represents a substituent selected from the group consisting of thiocyanate, cyano, amino sulfone derivatives, amido, substituted or not thioalkyl, hydroxylamine;
or a pharmaceutically acceptable salt, solvate, hydrate, polymorph, co-crystal thereof, for use as medicine.

15. Compounds according to claim 14 having general formula (V) 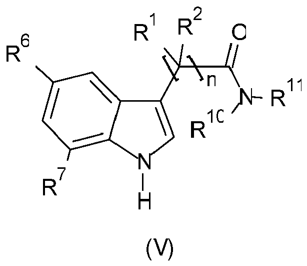 wherein n is an integer between 1 and 10;
R⁶ and R⁷ independently represent hydrogen, F, Br, Cl, CO₂H, CN, C₁-C₁₀ alkoxy; R¹ and R² represent independently in each of the n units a substituent selected from the group consisting of hydrogen and C₁-C₁₀ alkyl,
R¹⁰ and R¹¹ independently represent a substituent selected from the group consisting of hydrogen, substituted or not C₁-C₁₀ alkyl, C₂-C₁₀ alkenyl, C₂-C₁₀ alkynyl, C₆-C₁₈ aryl, heterocycle, or R¹⁰ and R¹¹ taken together with the nitrogen atom to which they are attached form a four to ten membered heterocycle;
or a pharmaceutically acceptable salt, solvate, hydrate, polymorph, co-crystal thereof, for use as medicine.
